(19) **Europäisches Patentamt** / **European Patent Office** / **Office européen des brevets**

(11) **EP 4 140 420 B1**

(12) # EUROPEAN PATENT SPECIFICATION

(45) Date of publication and mention
of the grant of the patent:
**02.04.2025 Bulletin 2025/14**

(21) Application number: **22202107.3**

(22) Date of filing: **17.08.2020**

(51) International Patent Classification (IPC):
*A61B 17/32* (2006.01)     *A61B 17/00* (2006.01)
*A61B 17/29* (2006.01)     *A61B 34/30* (2016.01)

(52) Cooperative Patent Classification (CPC):
**A61B 17/320092;** A61B 34/30; A61B 2017/003;
A61B 2017/00314; A61B 2017/00327;
A61B 2017/2908; A61B 2017/320071;
A61B 2017/320075; A61B 2017/320089;
A61B 2017/320094; A61B 2034/305;
A61B 2034/306

(54) **ULTRASONIC SURGICAL INSTRUMENT WITH A MULTI-PLANAR ARTICULATING SHAFT ASSEMBLY**

CHIRURGISCHES ULTRASCHALLINSTRUMENT MIT EINER MULTIPLANAREN GELENKSCHAFTANORDNUNG

INSTRUMENT CHIRURGICAL À ULTRASONS AVEC UN ENSEMBLE TIGE D'ARTICULATION MULTIPLANAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **30.08.2019   US 201916556661**
  **30.08.2019   US 201916556667**

(43) Date of publication of application:
**01.03.2023   Bulletin 2023/09**

(62) Document number(s) of the earlier application(s) in
accordance with Art. 76 EPC:
**20760927.2 / 4 021 318**

(73) Proprietor: **Cilag GmbH International
6300 Zug (CH)**

(72) Inventors:
• **REMM, Thomas B.
Loveland, 45140 (US)**
• **BLACK, Brian D.
Cincinnati, 45242 (US)**
• **HUNTER, Morgan R.
Cincinnati, 45242 (US)**
• **MUELLER, Karl W.
Cincinnati, 45242 (US)**

(74) Representative: **Carpmaels & Ransford LLP
One Southampton Row
London WC1B 5HA (GB)**

(56) References cited:
US-A- 6 063 098       US-A1- 2014 005 681
US-A1- 2014 005 702    US-A1- 2017 105 757
US-A1- 2019 059 931    US-B2- 9 095 367

EP 4 140 420 B1

## Description

### BACKGROUND

A variety of surgical instruments include an end effector having a blade element that vibrates at ultrasonic frequencies to cut and/or seal tissue (e.g., by denaturing proteins in tissue cells). These instruments include one or more piezoelectric elements that convert electrical power into ultrasonic vibrations, which are communicated along an acoustic waveguide to the blade element. The precision of cutting and coagulation may be controlled by the operator's technique and adjusting the power level, blade edge angle, tissue traction, and blade pressure. The power level used to drive the blade element may be varied (e.g., in real time) based on sensed parameters such as tissue impedance, tissue temperature, tissue thickness, and/or other factors. Some instruments have a clamp arm and clamp pad for grasping tissue with the blade element.

Such surgical instruments may be directly gripped and manipulated by a surgeon or incorporated into a robotically assisted surgery. During robotically assisted surgery, the surgeon typically operates a master controller to remotely control the motion of such surgical instruments at a surgical site. The controller may be separated from the patient by a significant distance (e.g., across the operating room, in a different room, or in a completely different building than the patient). Alternatively, a controller may be positioned quite near the patient in the operating room. Regardless, the controller typically includes one or more hand input devices (such as joysticks, exoskeletol gloves, master manipulators, or the like), which are coupled by a servo mechanism to the surgical instrument. In one example, a servo motor moves a manipulator supporting the surgical instrument based on the surgeon's manipulation of the hand input devices. During the surgery, the surgeon may employ, via a robotic surgical system, a variety of surgical instruments including an ultrasonic blade, a tissue grasper, a needle driver, an electrosurgical cautery probes, etc. Each of these structures performs functions for the surgeon, for example, cutting tissue, coagulating tissue, holding or driving a needle, grasping a blood vessel, dissecting tissue, or cauterizing tissue.

Document US 9,095,367 B2 discloses a surgical instrument comprising an articulable harmonic waveguide. The articulable harmonic waveguide comprises a first drive section comprising a proximal end and a distal end. The proximal end of the first drive section may be configured to connect to an ultrasonic transducer. The articulable harmonic waveguide further comprises a first flexible waveguide coupled to the distal end of the first drive section. An end effector extends distally from the first flexible waveguide. The surgical instrument further comprises an articulation actuator to flex the first flexible waveguide. **In** some examples, the articulable harmonic waveguides comprise a total curvature limiter. The total curvature limiter may comprise a mechanical or electrical break to prevent the articulable harmonic waveguide from exceeding one or more predetermined conditions. The mechanical breaks are formed on the waveguide.

Document US 2017/0105757 Al discloses articulating surgical instruments for treating tissue comprising an end effector and a shaft extending proximally from the end effector along a longitudinal axis. **In** certain examples, the shaft comprises a plurality of transverse spacer members as well as first and second rotatable members extending through at least a portion of the plurality of transverse spacer members. The first and second rotatable members may both be biased away from the longitudinal axis such that their respective directions of bias vary with rotation of the first rotatable member. When the respective directions of bias of the first and second rotatable members oppose one another, the shaft may be substantially straight. When the respective directions of bias of the first and second rotatable members are aligned with one another, the shaft may articulate away from the longitudinal axis in the direction of the alignment.

Document US 2014/0005681 Al discloses surgical instruments for use in handheld applications or with robotic surgical systems. The surgical instruments may comprise an end effector to treat tissue and a shaft extending proximally from the end effector along a longitudinal axis. Some embodiments may be usable with an instrument mounting portion of a robotic surgical instrument. For example, the shaft may extend proximally to the instrument mounting portion.

While several surgical instruments and systems have been made and used, it is believed that no one prior to the inventors has made or used the invention defined in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:

FIG. 1 depicts a front perspective view of a first example of an ultrasonic surgical instrument having an end effector, a first exemplary base assembly configured to connect to a robotic driven interface, and a first exemplary shaft assembly with a first exemplary acoustic waveguide;

FIG. 2 depicts a rear perspective view of the ultrasonic surgical instrument of FIG. 1;

FIG. 3A depicts an enlarged perspective view of the ultrasonic surgical instrument of FIG. 1 with the end effector in a closed position and the shaft assembly in a straight configuration;

FIG. 3B depicts the enlarged perspective view of the ultrasonic surgical instrument similar to FIG. 3A, but showing the end effector in an open position;

FIG. 4A depicts an enlarged perspective view of the ultrasonic surgical instrument of FIG. 1 with the end effector in a closed position and the shaft assembly in a first articulated configuration;

FIG. 4B depicts the enlarged perspective view of the ultrasonic surgical instrument similar to FIG. 4A, but with the shaft assembly in a second articulated configuration;

FIG. 5 depicts an enlarged perspective view of the ultrasonic surgical instrument of FIG. 1 with the base assembly having various components removed for greater clarity of an interior space of the base assembly;

FIG. 6 depicts an enlarged front view of the ultrasonic surgical instrument of FIG. 1 with the base assembly having various components removed for greater clarity of the interior space of the base assembly;

FIG. 7 depicts a front perspective view of a second example of an ultrasonic surgical instrument having a multi-planar shaft assembly in a straight configuration;

FIG. 8 depicts an enlarged, front perspective view of the shaft assembly of FIG. 7 with a distal articulation section and a proximal articulation section respectively in a distal articulated configuration and a proximal articulated configuration;

FIG. 9 depicts an enlarged, rear perspective view of the distal articulation section of FIG. 8 in the straight configuration;

FIG. 10 depicts an enlarged, front perspective view of the distal articulation section of FIG. 8 in the straight configuration;

FIG. 11 depicts a sectional perspective view of the distal articulation section of FIG. 10 taken along section line 11-11 of FIG. 10 and having various components removed for additional clarity;

FIG. 12 depicts an enlarged, front view of the proximal articulation section of FIG. 8 in the proximal articulated configuration;

FIG. 13 depicts a rear, distal perspective view of a distal link of the proximal articulation section of FIG. 8;

FIG. 14 depicts a rear, proximal perspective view of the distal link of FIG. 13;

FIG. 15 depicts a distal end elevational view of the distal link of FIG. 13;

FIG. 16 depicts a proximal end elevational view of the distal link of FIG. 13;

FIG. 17 depicts a rear, distal perspective view of an intermediate link of the proximal articulation section of FIG. 8;

FIG. 18 depicts a rear, proximal perspective view of the intermediate link of FIG. 17;

FIG. 19 depicts a distal end elevational view of the intermediate link of FIG. 17;

FIG. 20 depicts a proximal end elevational view of the intermediate link of FIG. 17;

FIG. 21 depicts a rear, distal perspective view of a proximal link of the proximal articulation section of FIG. 8;

FIG. 22 depicts a rear, proximal perspective view of the proximal link of FIG. 21;

FIG. 23 depicts a distal end elevational view of the proximal link of FIG. 21;

FIG. 24 depicts a proximal end elevational view of the proximal link of FIG. 21;

FIG. 25 a front perspective view of a first exemplary multi-flex acoustic waveguide having a flexible distal yaw ribbon and a flexible proximal pitch ribbon in a straight contour;

FIG. 26 depicts a top view of the acoustic waveguide of FIG. 25 in the straight contour;

FIG. 27 depicts a top view of a second exemplary multi-flex acoustic waveguide with a flexible distal yaw ribbon and a flexible proximal pitch ribbon in an exemplary dual arcuate contour;

FIG. 28 depicts a front perspective view of a third exemplary multi-flex acoustic waveguide having a first example of a flexible wire in a straight contour;

FIG. 29 depicts a cross-sectional view of the acoustic waveguide of FIG. 28 taken along section line 29-29 of FIG. 28;

FIG. 30A depicts a top view of the acoustic waveguide of FIG. 28 with the flexible wire in the straight contour;

FIG. 30B depicts the top view of the acoustic waveguide similar to FIG. 30A, but with the flexible wire in an exemplary arcuate contour;

FIG. 31 depicts a front perspective view of a fourth exemplary multi-flex acoustic waveguide having a second example of a flexible wire in a straight contour

FIG. 32 depicts a cross-sectional view of the acoustic waveguide of FIG. 31 taken along section line 32-32 of FIG. 31;

FIG. 33 depicts a front perspective view of a fifth exemplary multi-flex acoustic waveguide having a third example of a flexible wire in a straight contour;

FIG. 34 depicts a cross-sectional view of the acoustic waveguide of FIG. 33 taken along section line 34-34 of FIG. 33;

FIG. 35A depicts a top view of a sixth exemplary multi-flex acoustic waveguide with a flexible waveguide body in the straight contour;

FIG. 35B depicts the top view of the acoustic waveguide similar to FIG. 35A, but with the flexible waveguide body in an exemplary arcuate contour;

FIG. 36 depicts a perspective view of an ultrasonic blade with a first example of a circumferential blade profile having a first backcutting edge;

FIG. 37 depicts a distal end view of the ultrasonic blade of FIG. 36;

FIG. 38 depicts a perspective view of another ultrasonic blade with a second example of a circumferential blade profile having a second backcutting edge;

FIG. 39 depicts a distal end view of the ultrasonic blade of FIG. 38;

FIG. 40A depicts an enlarged, sectional, perspective view of the ultrasonic surgical instrument of FIG. 1 taken along a centerline thereof showing an ultrasonic blade of the end effector positioned relative to a clamp arm of the end effector with the shaft assembly in the straight configuration;

FIG. 40B depicts an enlarged, cross-sectional view of the ultrasonic surgical instrument of FIG. 40A taken along a centerline thereof showing the ultrasonic blade of the end effector positioned relative to the clamp arm of the end effector with the shaft assembly in the first articulated configuration;

FIG. 41A depicts an enlarged, cross-sectional view of the end effector and the shaft assembly taken along a centerline thereof showing the ultrasonic blade positioned relative to the clamp arm with the shaft assembly in the straight configuration;

FIG. 41B depicts the enlarged, cross-sectional view of the end effector and the shaft assembly similar to FIG. 41A, but showing the ultrasonic blade positioned relative to the clamp arm with the shaft assembly in the first articulated configuration;

FIG. 42 depicts an enlarged, cross-sectional view of a third example of an ultrasonic surgical instrument taken along a centerline thereof with the end effector of FIG. 1 and a second exemplary shaft assembly having the ultrasonic blade fixed relative to the clamp arm in the straight configuration and the articulated configuration;

FIG. 43A depicts an enlarged, perspective view of a second exemplary base assembly of the ultrasonic surgical instrument of FIG. 42 having various components removed for greater clarity of a passively shiftable transducer assembly in a proximal position while the shaft assembly of FIG. 42 is in the straight configuration;

FIG. 43B depicts the enlarged, perspective view of the base assembly similar to FIG. 43A, but showing the passively shiftable transducer assembly in a distal position while the shaft assembly of FIG. 42 is in the articulated configuration;

FIG. 44A depicts an enlarged perspective view of a fourth example of an ultrasonic surgical instrument with a third exemplary base assembly having various components removed for greater clarity of an actively shiftable transducer assembly in a proximal position; and

FIG. 44B depicts the enlarged, perspective view of the base assembly similar to FIG. 44A, but showing the actively shiftable transducer assembly in a distal position.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "front," "rear," "clockwise," "counterclockwise," "longitudinal," and "transverse" also are used herein for reference to relative positions and directions. Such terms are used below with reference to views as illustrated for clarity and are not intended to limit the invention described herein.

I. Exemplary Surgical Instrument

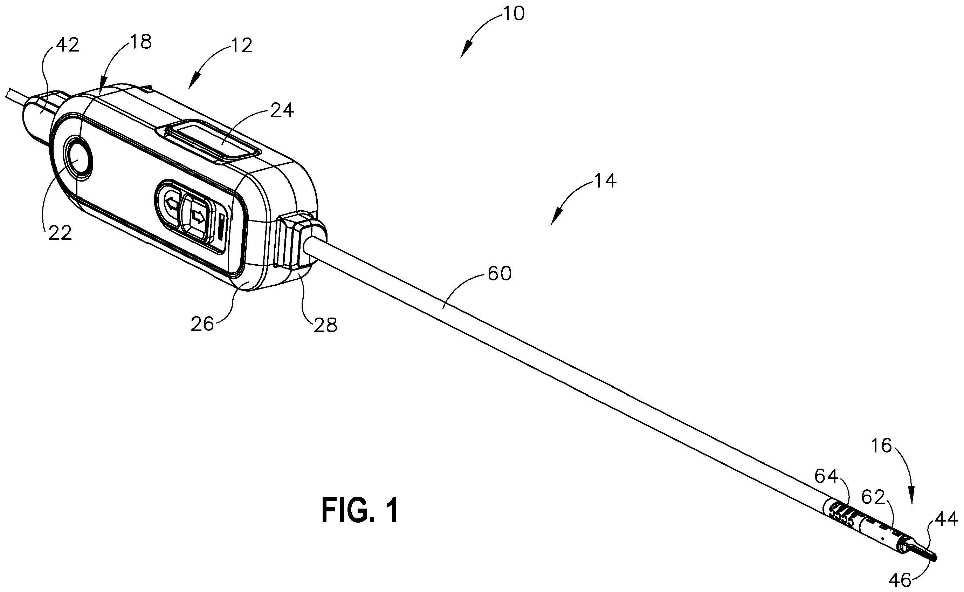

FIG. 1 shows an exemplary surgical instrument, such as an ultrasonic surgical instrument (10). At least part of ultrasonic surgical instrument (10) may be constructed and operable in accordance with at least some of the teachings of any of the various patents, patent application publications, and patent applications that are cited herein. As described therein and as will be described in greater detail below, ultrasonic surgical instrument (10) is operable to cut tissue and seal or weld tissue (e.g., a blood vessel, etc.) substantially **simultaneously.**

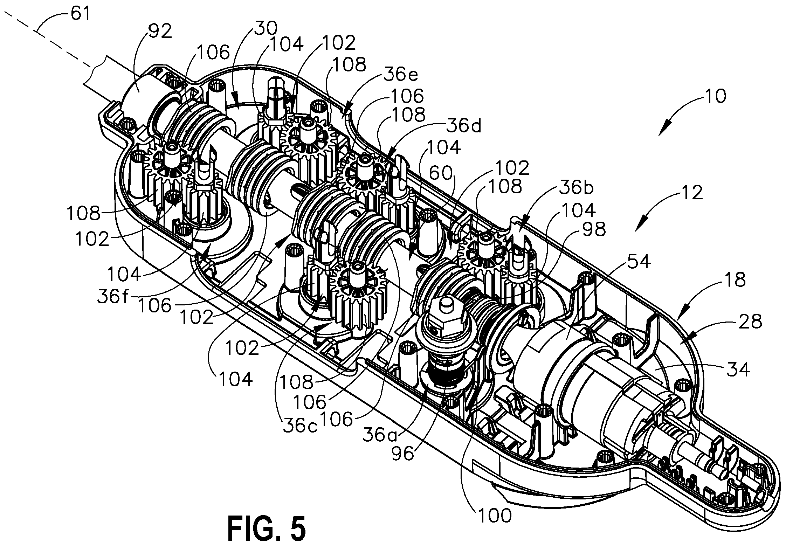

Ultrasonic surgical instrument (10) of the present example comprises a body assembly, such as a first exemplary base assembly (12), a shaft assembly (14), and an end effector (16). Base assembly (12) includes a housing (18), a button (22), and a pair of latch clasps (24). Button (22) is operatively connected to an electrical base power controller (not shown) and configured to selectively power ultrasonic surgical instrument (10) for use. In addition, housing (18) of the present example includes a front housing cover (26) and a rear housing cover (28) removably secured together via latch clasps (24). More particularly, latch clasps (24) removably secure front housing cover (26) to rear housing cover (28) such that front housing cover (26) may be removed for accessing an interior space (30) (*see* FIG. 5) within base assembly (12). Shaft assembly (14) distally extends from base assembly (12) to end effector (16) to thereby communicate mechanical and/or electrical forces therebetween for use as will be discussed below in greater detail. As shown in the present example, base assembly (12) is configured to operatively connect to a robotic drive (not shown) for driving various features of shaft assembly (14) and/or end effector (16). However, in another example, body assembly may alternatively include a handle assembly (not shown), which may include a pistol grip (not shown) in one example, configured to be directly gripped and manipulated by the surgeon for driving various features of shaft assembly (14) and/or end effector (16). The invention is thus not intended to be unnecessarily limited to use with base assembly (12) and the robotic drive (not shown).

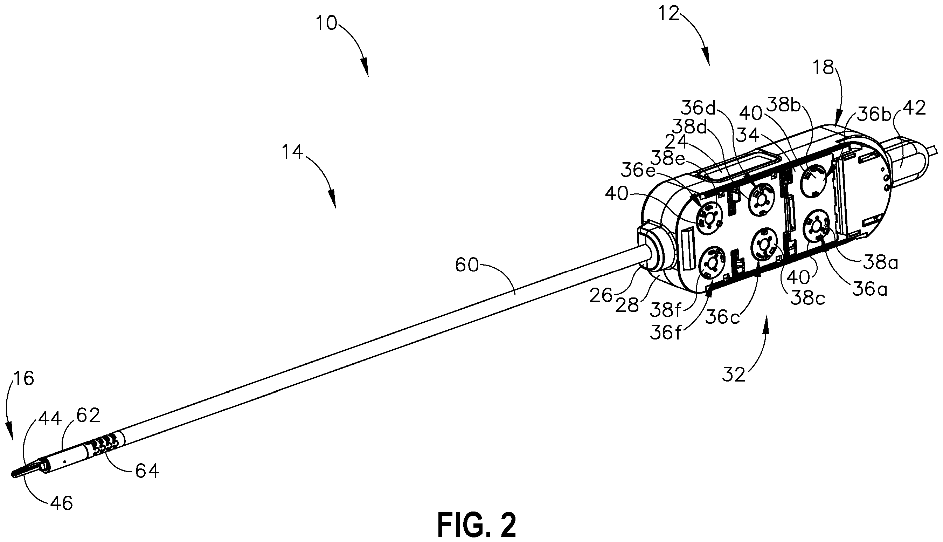

To this end, with respect to FIG. 2, base assembly (12) includes a robotic driven interface (32) extending through a base plate (34) of rear housing cover (28) and configured to mechanically couple with the robotic drive (not shown). Robotic driven interface (32) of the present example includes a plurality of instrument actuators (36a, 36b, 36c, 36d, 36e, 36f) having a plurality of input bodies (38a, 38b, 38c, 38d, 38e, 38f), respectively. Each input body (38a, 38b, 38c, 38d, 38e, 38f), which may also be referred to herein as a "puck," is configured to removably connect with the robotic drive (not shown) and, in the present example, is generally cylindrical and rotatable about an axis. Input bodies (38a, 38b, 38c, 38d, 38e, 38f) have a plurality of slots (40) configured to receive portions of the robotic drive (not shown) for gripping and rotatably driving input bodies (38a, 38b, 38c, 38d, 38e, 38f) in order to direct operation of shaft assembly (14) and/or end effector (16) as will be discussed below in greater detail. Base assembly (12) also receives an electrical plug (42) operatively connected to an electrical power source (not shown) to provide electrical power to base assembly (12) for operation as desired, such as powering electrical base power controller (not shown) and directing electrical energy to various features of shaft assembly (14) or end effector (16) associated with cutting, sealing, or welding tissue.

A. Exemplary End Effector and Acoustic Drivetrain

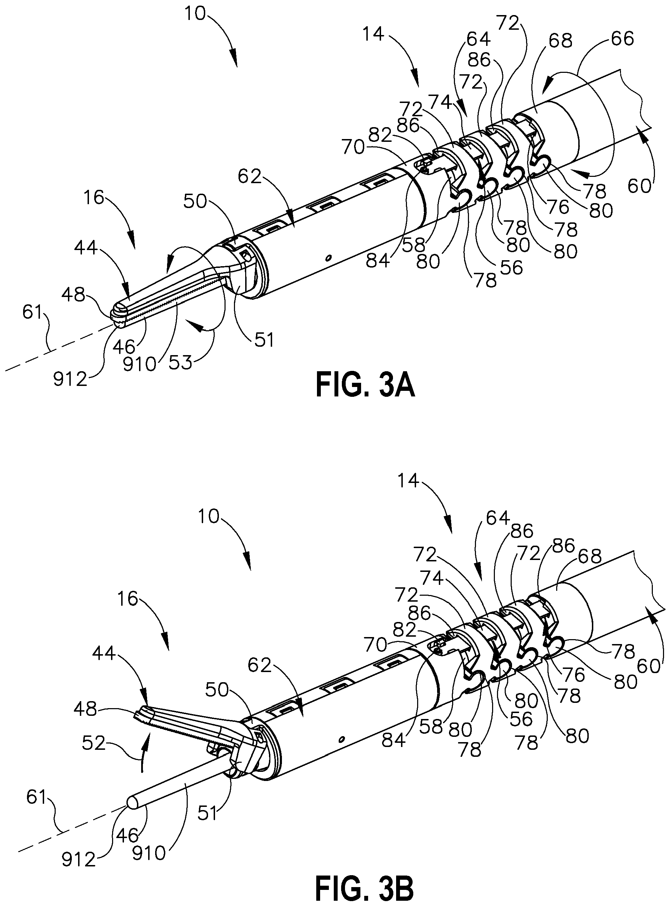

As best seen in FIGS. 3A-3B, end effector (16) of the present example includes a clamp arm (44) and an ultrasonic blade (46). Clamp arm (44) has a clamp pad (48) secured to an underside of clamp arm (44), facing blade (46). In one example, clamp pad (48) may comprise polytetrafluoroethylene (PTFE) and/or any other suitable material(s). Clamp arm (44) is pivotally secured to a distally projecting tongue (50) of shaft assembly (14). Clamp arm (44) is operable to selectively pivot toward and away from blade (46) to selectively clamp tissue between clamp arm (44) and blade (46). A pair of arms (51) extend transversely from clamp arm (44) and are pivotally secured to another portion of shaft assembly (14) configured to longitudinally slide to pivot clamp arm (44) as indicated by an arrow (52) between a closed position shown in FIG. 3A and an open position shown in FIG. 3B.

In addition to pivoting relative to blade (46), clamp arm (44) of the present example is further configured to rotate about blade (46) relative to blade (46) and also relative to shaft assembly (14) as indicated by an arrow (53). In one example, clamp arm (44) rotates in the clockwise or counterclockwise directions completely around blade (46) and may be selectively fixed in any angular position relative to blade (46) for directing clamp arm (44) from the open position to the closed position for clamping tissue. In another example, clamp arm (44) may have rotational stops (not shown) configured to limit rotational movement of clamp arm (44) relative to blade (46) in one or more predetermined positions.

Blade (46) of the present example is operable to vibrate at ultrasonic frequencies in order to effectively cut through and seal tissue, particularly when the tissue is being compressed between clamp pad (48) and blade (46). Blade (46) is positioned at a distal end of an acoustic drivetrain. This acoustic drivetrain includes a transducer assembly (54) (*see* FIG. 5) and an acoustic waveguide (56), which includes a flexible portion (58) discussed below in greater detail. It should be understood that waveguide (56) may be configured to amplify mechanical vibrations transmitted through waveguide (56). Furthermore, waveguide (56) may include features operable to control the gain of the longitudinal vibrations along waveguide (56) and/or features to tune waveguide (56) to the resonant frequency of the system. Various suitable ways in which waveguide (56) may be mechanically and acoustically coupled with transducer assembly (54) (*see* FIG. 5) will be apparent to those of ordinary skill in the art in view of the teachings herein.

Those of ordinary skill in the art will understand that, as a matter of physics, a distal end of blade (46) is located at a position corresponding to an antinode associated with resonant ultrasonic vibrations communicated through flexible portion (58) of waveguide (56). When transducer assembly (54) *(see* FIG. 5) is energized, the distal end of blade (46) is configured to move longitudinally in the range of, for example, approximately 10 to 500 micrometers peak-to-peak, and in some instances in the range of about 20 to about 200 micrometers at a predetermined vibratory frequency $f_o$ of, for example, 55.5 kHz. When transducer assembly (54) *(see* FIG. 5) of the present example is activated, these mechanical oscillations are transmitted through waveguide (56) to reach blade (46), thereby providing oscillation of blade (46) at the resonant ultrasonic frequency. Thus, when tissue is secured between blade (46) and clamp pad (48), the ultrasonic oscillation of blade (46) may simultaneously sever the tissue and denature the proteins in adjacent tissue cells, thereby providing a coagulative effect with relatively little thermal spread. In some versions, end effector (16) is operable to apply radiofrequency (RF) electrosurgical energy to tissue in addition to applying ultrasonic energy to tissue. In any case, other suitable configurations for an acoustic transmission assembly and transducer assembly (54) will be apparent to one of ordinary skill in the art in view of the teachings herein. Similarly, other suitable configurations for end effector (16) will be apparent to those of ordinary skill in the art in view of the teachings herein.

B. Exemplary Shaft Assembly and Articulation Section

As shown in FIGS. 3A-3B, shaft assembly (14) includes a proximal shaft portion (60) extending along a longitudinal axis (61), a distal shaft portion (62) distally projecting relative to the proximal shaft portion (60), and an articulation section (64) extending between proximal and distal shaft portions (60, 62). Shaft assembly (14) is configured to rotate about longitudinal axis (61) as indicated by an arrow (66). In one example, shaft assembly (14) rotates in the clockwise or counterclockwise directions completely around longitudinal axis (61) and may be selectively fixed in any rotational position about longitudinal axis (61) for positioning articulation section (64) and/or end effector (16) about longitudinal axis (61). While end effector (16) generally rotates with shaft assembly (14) as indicated by arrow (66), end effector (16) may be simultaneously and independently rotated as indicated by arrow (53) relative to shaft assembly (14) during use for repositioning portions of shaft assembly (14) and/or end effector (16) as desired.

Articulation section (64) is configured to selectively position end effector (16) at various lateral deflection angles relative to longitudinal axis (61) defined by proximal shaft portion (60). Articulation section (64) may take a variety of forms. In the present example, articulation section (64) includes a proximal link (68), a distal link (70), and a plurality of intermediate links (72) connected in series between proximal and distal links (68, 70). Articulation section (64) further includes a pair of articulation bands (74) extending along a pair of respective channels (76) collectively defined through links (68, 70, 72). Links (68, 70, 72) are generally configured to pivot relative to each other upon actuation of articulation bands (74) to thereby bend articulation section (64) with flexible portion (58) of waveguide (56) therein to achieve an articulated state. By way of example only, articulation section (64) may alternatively or additionally be configured in accordance with one or more teachings of U.S. Pat. No. 9,402,682, entitled "Articulation Joint Features for Articulating Surgical Device," issued August 2, 2016, the disclosure of which is referred to herein. As another merely illustrative example, articulation section (64) may alternatively or additionally be configured in accordance with one or more teachings of U.S. Pat. No. 9,393,037, issued July 19, 2016, entitled "Surgical Instruments with Articulating Shafts," the disclosure of which is referred to herein and U.S. Pat. No. 9,095,367, issued August 4, 2015, entitled "Flexible Harmonic Waveguides/Blades for Surgical Instruments," the disclosure of which is referred to herein. In addition to or in lieu of the foregoing, articulation section (64) and/or may be constructed and/or operable in accordance with at least some of the teachings of U.S. Pat. No. 10,034,683, entitled "Ultrasonic Surgical Instrument with Rigidizing Articulation Drive Members," issued on July 31, 2018. Alternatively, articulation section (64) may be constructed and/or operable in any other suitable fashion.

Links (68, 70, 72) shown in FIGS. 3B-4B pivotally interlock to secure distal shaft portion (62) relative to proximal shaft portion (60) while allowing for deflection of distal shaft portion (62) relative to longitudinal axis (61). In the present example, proximal link (68) is rigidly connected to proximal shaft portion (60) and has a pair of arcuate grooves (78) opposed from each other. Intermediate links (72) respectively have a pair of arcuate tongues (80) proximally extending therefrom and a pair of arcuate grooves (78) positioned distally opposite from respective tongues (80). Each intermediate link (72) has tongues (80) pivotally received within adjacent arcuate grooves (78) of another intermediate link (72) or proximal link (68) as applicable. Distal link (70) is rigidly connected to distal shaft portion (62) and has another pair of arcuate tongues (80) opposed from each other and pivotally received within adjacent arcuate grooves (78) of intermediate link (72). Tongues (80) and grooves (78) connect together to form the series of interlocked links (68, 70, 72).

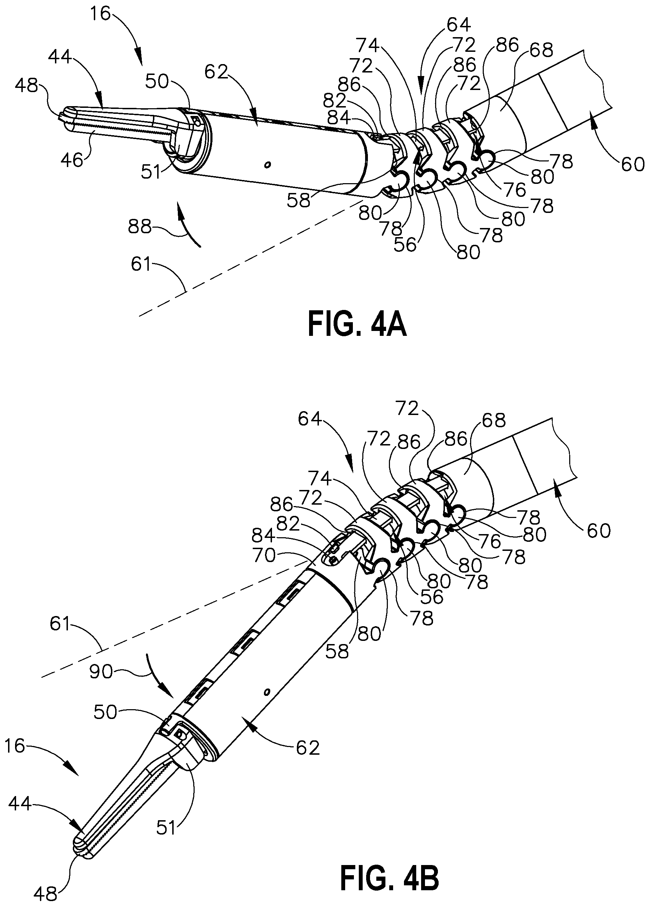

Distal link (70) further includes a pair of opposing notches (82) with a pin (84) therein configured to receive distal end portions of respective articulation bands (74). More particularly, pins (84) extend through a hole in each respective articulation bands (74) while distal end portions of respective articulation bands (74) are coupled within notches (82). Slots (86) in each of intermediate and proximal links (72, 68) longitudinally align with each other and notches (82) to collectively define channels (76) configured to receive articulation bands (74) while allowing articulation bands (74) to slide relative to links (68, 70, 72). To this end, when articulation bands (74) translate longitudinally in an opposing fashion, this will cause articulation section (64) to bend, thereby laterally deflecting end effector (16) away from the longitudinal axis (61) of proximal shaft portion (60) from a straight configuration as shown in FIG. 3B to a first articulated configuration as shown in FIG. 4A and indicated by an arrow (88) or a second articulated configuration as shown in FIG. 4B and indicated by an arrow (90). In particular, end effector (16) will be articulated toward the articulation band (74) that is being pulled proximally. During such articulation, the other articulation band (74) may be pulled distally. Alternatively, the other articulation band (74) may be driven distally by an articulation control. Furthermore, flexible acoustic waveguide (56) is configured to effectively communicate ultrasonic vibrations from waveguide (56) to blade (46) even when articulation section (64) is in an articulated configuration as shown in FIGS. 4A-4B.

C. Exemplary Base Assembly with Instrument Actuators for Robotic Interface

FIG. 5 shows interior space (30) of base assembly (12) with instrument actuators (36a, 36b, 36c, 36d, 36e, 36f) in greater detail. Generally, instrument actuators (36a, 36b, 36c, 36d, 36e, 36f) are engaged with shaft assembly (14) and configured to direct movement of end effector (16) and/or shaft assembly (14), such as movement indicated above in one example by arrows (52, 53, 66, 88, 90) (see FIGS. 3A-4B). Shaft assembly (14) is received within base assembly (12) and supported by bearings (92) therein to operatively connect each respective instrument actuator (36a, 36b, 36c, 36d, 36e, 36f) to shaft assembly (14) as well as operatively connect acoustic waveguide (56) (see FIG. 3A) to transducer assembly (54) and a generator (not shown) of the acoustic drivetrain. More particularly, transducer assembly (54) is coupled with generator (not shown) such that transducer assembly (54) receives electrical power from generator (not shown). Piezoelectric elements (not shown) in transducer assembly (54) convert that electrical power into ultrasonic vibrations. Generator (not shown) may be coupled to the electrical power source (not shown) via electrical plug (42) (see FIG. 1) and a control module (not shown) that are configured to provide a power profile to transducer assembly (54) that is particularly suited for the generation of ultrasonic vibrations through transducer assembly (54). By way of example only, generator (not shown) may comprise a GEN04 or GEN11 sold by Ethicon Endo-Surgery, Inc. of Cincinnati, Ohio. In addition or in the alternative, generator (not shown) may be constructed in accordance with at least some of the teachings of U.S. Pub. No.

2011/0087212, entitled "Surgical Generator for Ultrasonic and Electrosurgical Devices," published April 14, 2011, the disclosure of which is referred to herein. Still other suitable forms that generator (not shown) may take, as well as various features and operabilities that generator (not shown) may provide, will be apparent to those of ordinary skill in the art in view of the teachings herein.

The present example of base assembly (12) shown in FIGS. 5-6 includes six instrument actuators (36a, 36b, 36c, 36d, 36e, 36f), although it will be appreciated that any such number of such instrument actuators (36a, 36b, 36c, 36d, 36e, 36f) configured to direct movement of shaft assembly (14) and/or end effector (16) may be similarly used. As shown with respect to operation of ultrasonic surgical instrument (10), instrument actuator (36a) is more particularly a roll system actuator (36a) configured to rotate shaft assembly (14) about longitudinal axis (61). In contrast, instrument actuators (36b, 36c, 36d, 36e, 36f) are linear system actuators (36b, 36c, 36d, 36e, 36f) configured to translationally drive movement of portions of end effector (16) and/or shaft assembly (14) while simultaneously allowing for rotation of shaft assembly (14) via roll system actuator (36a).

Roll system actuator (36a) in one example includes a drive spool (96) rigidly connected to puck (38a) (see FIG. 2) and a driven spool (98) rigidly connected to proximal shaft portion (60) within housing (18). Drive spool (96) is mounted to rotate with puck (38a) (see FIG. 2) about a common puck axis, whereas driven spool (98) is mounted to rotate with proximal shaft portion (60) about the longitudinal axis (61). A cable (100) wraps around each of the drive and driven spools (96, 98), accommodating the differing orientation of the puck axis and longitudinal axis (61), such that rotating drive spool (96) via puck (38a) (see FIG. 2) urges rotation of driven spool (98). In turn, shaft assembly (14), including proximal and distal shaft portions (60, 62) rotates about longitudinal axis (61) as indicated by arrow (66) (see FIG. 3A), such as by robotically driven actuation of puck (38a) (see FIG. 2).

Linear system actuators (36b, 36c, 36d, 36e, 36f) of the present example include a gear-rack mechanism (102) having a rotatable drive gear (104), a translatable rack gear (106), and an idler gear (108) connected therebetween. Drive gears (104) are respectively connected to and rigidly project from pucks (38b, 38c, 38d, 38e, 38f) (see FIG. 2), whereas each rack gear (106) is connected to another portion of proximal shaft portion (60) directing movement of shaft assembly (14) and/or end effector (16) as discussed above. Each rack gear (106) is cylindrical and rigidly connected relative to proximal shaft portion (60) to rotate therewith. Rack gear (106) is thereby configured to rotate with shaft assembly (14) while remaining meshed with idler gear (108). Rotating respective pucks (38b, 38c, 38d, 38e, 38f) (see FIG. 2) thus respectively rotates drive gears (104) and idler gears (108) to translate rack gears (106) as desired.

In the present example, with respect to FIGS.

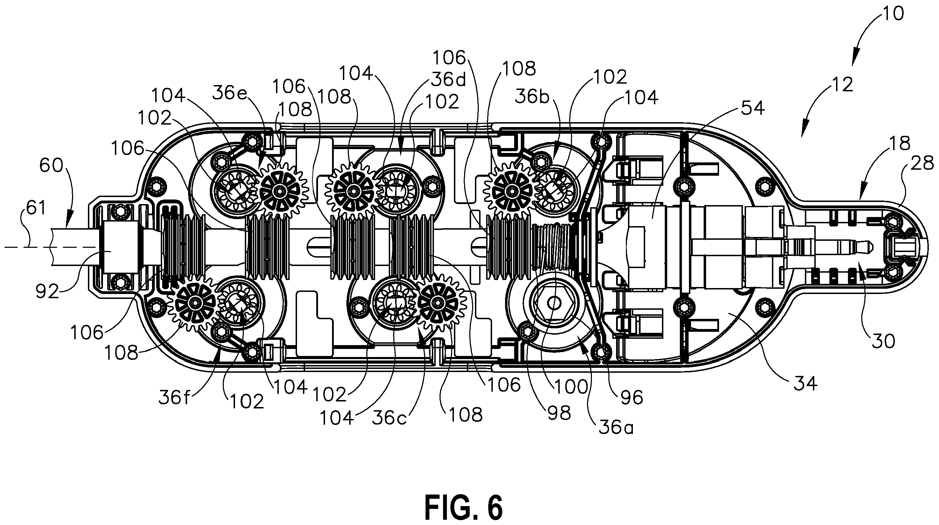

2-4B and FIG. 6, linear system actuator (36b) has puck (38b) operatively connected to clamp arm (44) to direct movement of clamp arm (44) between the open and closed positions according to arrow (52). Linear system actuators (36c, 36d) have respective pucks (38c, 38d) operatively connected to clamp arm (44) to direct movement of clamp arm (44) around blade (46) in both the clockwise and counterclockwise directions according to arrow (53). In addition, linear system actuators (36e, 36f) have respective pucks (38e, 38f) operatively connected to articulation bands (74) to direct movement of articulation section (64) according to arrows (88, 90) for deflecting end effector (16) relative to longitudinal axis (61). Of course, in other examples, instrument actuators (36a, 36b, 36c, 36d, 36e, 36f) may be alternatively configured with more or less actuators (36a, 36b, 36c, 36d, 36e, 36f) and/or more or less movement as desired. The disclosure is thus not intended to be unnecessarily limited to instrument actuators (36a, 36b, 36c, 36d, 36e, 36f) or particular movements of shaft assembly (14) and/or end effector (16) as described in the present example.

II. Exemplary Multi-Planar Articulation of Shaft Assembly

In some instances, with respect to FIGS. 1-4B, it may be desirable to guide deflection of end effector (16) at least in part according to various properties and/or constraints associated with components passing through articulation section (64) during use. By way of example, greater variability in such deflection, such as by increased articulation along shaft assembly (14), may increase strain on one or more flexible components within articulation section (64). Articulation section (64) may thus be desirably articulated via links (68, 70, 72) in one example to accurately and precisely guide movement of flexible components within articulation section (64) while reducing strain that may otherwise occur through these flexible components, such as acoustic waveguide (56).

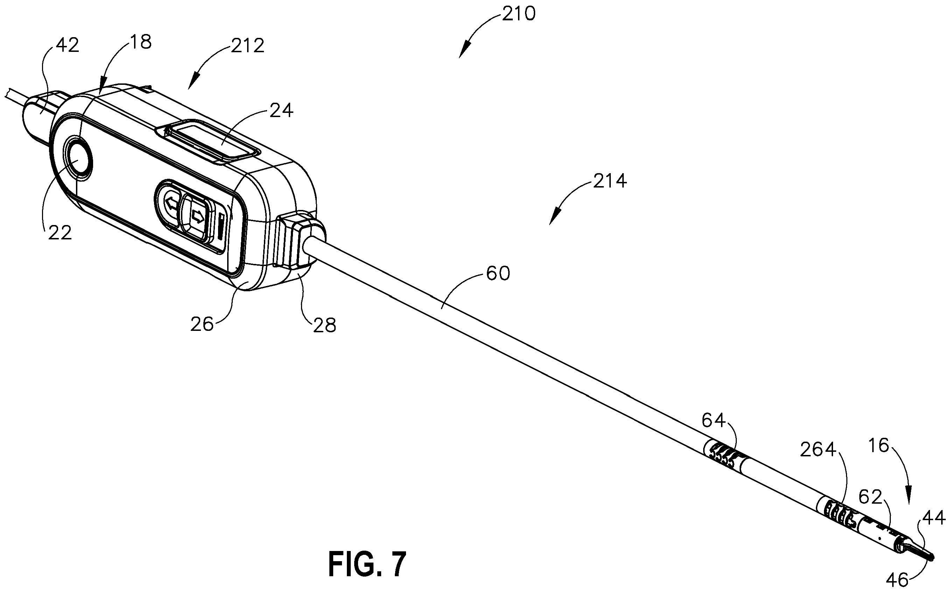

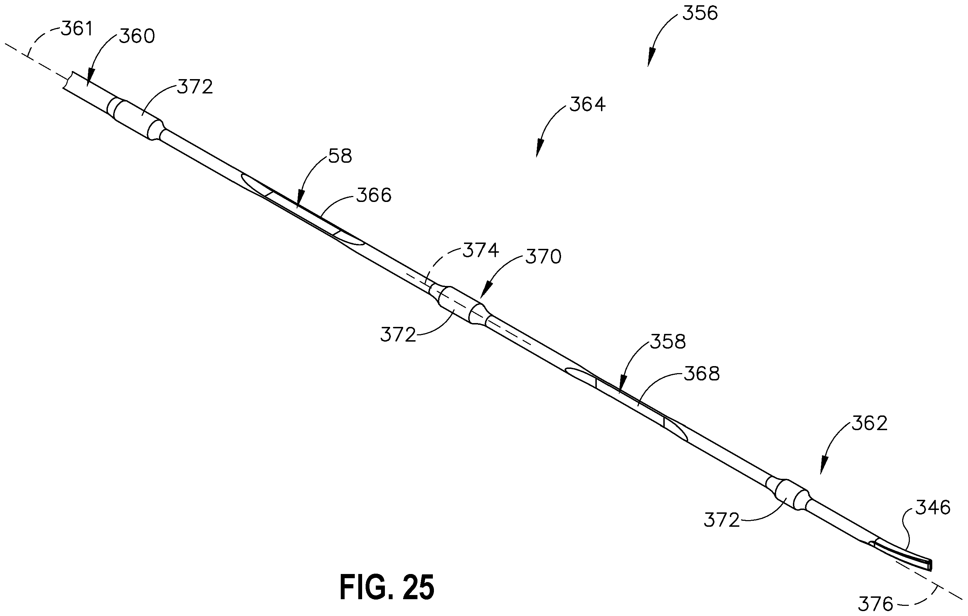

By way of further example, greater variability of deflection along shaft assembly (14) may incorporate a plurality of articulation sections (64) with respective links (68, 70, 72) for guiding any one of a plurality of acoustic waveguides (356, 456, 556, 656, 756, *856*) (*see* FIGS. 25-35B) through greater degrees of freedom than acoustic waveguide (56) of ultrasonic surgical instrument (10). To this end, shaft assembly (14) with end effector (16) is more generally configured to move longitudinally along longitudinal axis (61), laterally perpendicular to longitudinal axis (61), and transversely perpendicular to longitudinal axis (61) as well as rotate end effector (16) about longitudinal axis (61) and pivot end effector (16) along a plane, which may be pitch or yaw depending on the relative position of end effector (16). While such movement provides five degrees of freedom to end effector (16) via acoustic waveguide (56) during use, any one or more of the plurality of acoustic waveguides (356, 456, 556, 656, 756, 856) (*see* FIGS. 25-35B) described below are configured to enable end effector (16) to pivot through an additional plane for six degrees of freedom. Additional articulation sections (64) and/or alternative articulations (not shown) are thus configured to guide deflection of end effector (16) while reducing strain on acoustic waveguides (356, 456, 556, 656, 756, 856) (*see* FIGS. 25-35B). While the following provides additional details with respect to a second example of an ultrasonic surgical instrument (210) having dual articulation sections (64, 164) as shown in FIGS. 7-8, the disclosure is not intended to be unnecessarily limited to one or more of such articulation sections (64, 164). Indeed, any alternative articulation section (not shown) may be used alone or in combination for supporting acoustic waveguides having one or more flexible portions, such as acoustic waveguides (356, 456, 556, 656, 756, 856) (*see* FIGS. 25-35B) described below in greater detail. In addition, like numbers below indicate like features described above in greater detail.

A. Articulation Section for Multi-Planar Articulation

FIGS. 7-8 show a second example of an ultrasonic surgical instrument (210) having another example of a base assembly (212) and a distally extending multi-planar shaft assembly (214) with end effector (16). Base and shaft assemblies (212, 214) are similar to base and shaft assemblies (12, 14) (*see* FIG. 1) discussed above in greater detail, but are collectively configured for multi-planar articulation. More particularly, shaft assembly (214) includes articulation section (64) as a proximal articulation section (64) and further includes a distal articulation section (264). Base assembly (212) is thus configured to direct articulation of proximal articulation section (64) as discussed above with respect to base assembly (12) (*see* FIG. 1) and also configured to direct articulation of distal articulation section (264). Such movement of distal articulation section (264) in one example is performed by an additional instrument actuator (not shown). Alternatively, movement of distal articulation section (64) in another example is performed by another one of instrument actuators (36a, 36b, 36c, 36d, 36e, 36f). Unless explicitly stated herein, base and shaft assemblies (212, 214) are otherwise constructed and operable as base and shaft assemblies (12, 14) (*see* FIG. 1) discussed above in greater detail.

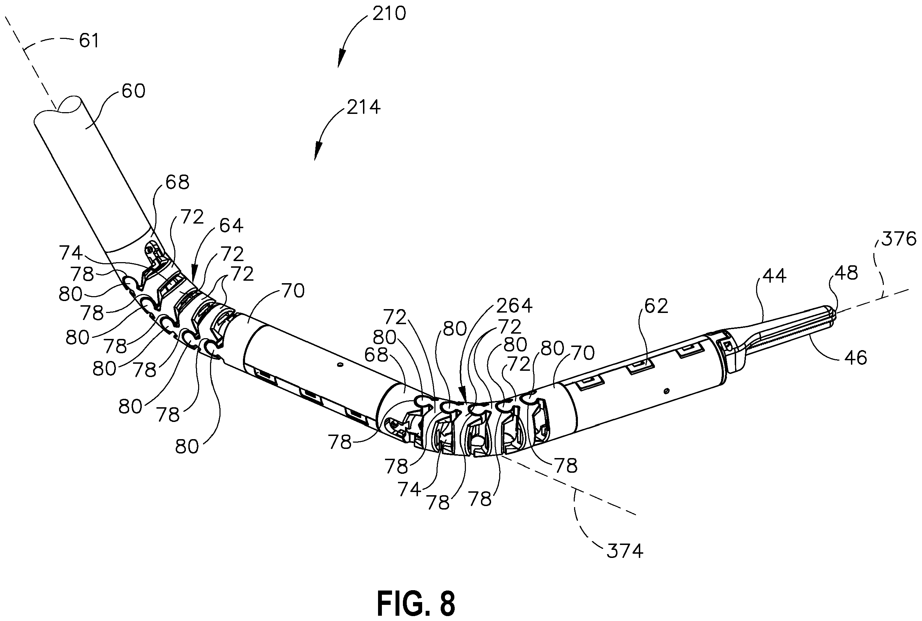

Proximal and distal articulation sections (64, 264) are similarly constructed in the present example with links (68, 70, 72) as discussed above. Proximal articulation section (64) thus articulates through a plane, whereas distal articulation section (264) articulates through another plane. In the present example, these planes are perpendicular to each other. Given the rotational orientation of shaft assembly (214) as shown in FIGS. 7-8, proximal articulation section (64) articulates through a pitch plane and distal articulation section (264) articulates through a yaw plane relative to clamp arm (44). However, it will be appreciated that such planes change relative to clamp arm (44) and/or as oriented in FIGS. 7-8, such as the disclosure is not intended to be unnecessarily limited to the yaw and pitch planes as shown in the present example. While FIG. 8 shows one example of dual articulation for each of proximal and distal articulation sections (64, 264) such that end effector (16) may be selectively moved according to six degrees of freedom, it will be further appreciated that any desired articulation and combination of respective articulations may be similarly used. Again, the disclosure is not intended to be unnecessarily limited to the particular angles of articulation shown in the yaw and pitch planes of the present example.

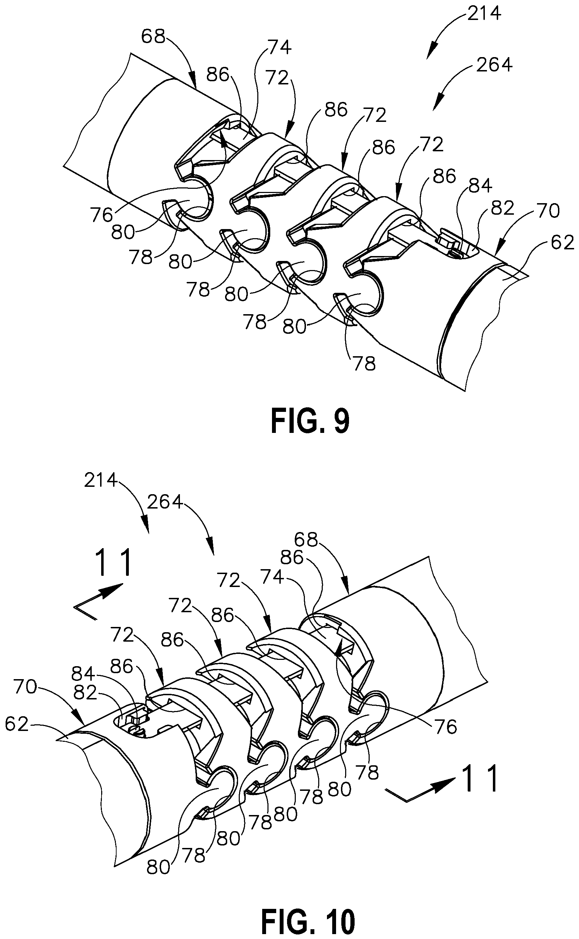

FIGS. 9-12 show distal articulation section (264) with proximal, distal, and intermediate links (68, 70, 72), articulation bands (74), and a distal flexible portion (358) of a first exemplary multi-flex acoustic waveguide (356) extending therethrough. Links (68, 70, 72) collectively define channels (76) configured to receive articulation bands (74) such that articulation bands (74) transversely align links (68, 70, 72) with a remainder of shaft assembly (214) as well as provide transverse support of links (68, 70, 72) along distal articulation section (264). As discussed above, links (68, 70, 72) have arcuate grooves (78) receiving arcuate tongues (80) along a lateral centerline positioned between articulation bands (74) such that articulation bands (74) are transversely offset and on opposing sides of distal flexible portion (358) thereby maintaining axial position of distal articulation section (264). Furthermore, each link (68, 70, 72) defines a link hollow (266) configured to receive distal flexible portion (358) and provide distal flexible portion (358) with sufficient and constant clearance space therealong to remain untouched by any portion of one of links (68, 70, 72) whether in the straight configuration or any articulated configuration, which is limited to maximum articulated configurations via cooperating distal and proximal stops (268, 270). To this end, proximal stop (270) on one link (68, 70, 72) is configured to engage distal stop (268) on another adjacent link (68, 70, 72) to thereby limit collective articulation of distal articulation section (264) and, in turn, limit strain due to articulation on distal flexible portion (358) of acoustic waveguide (356).

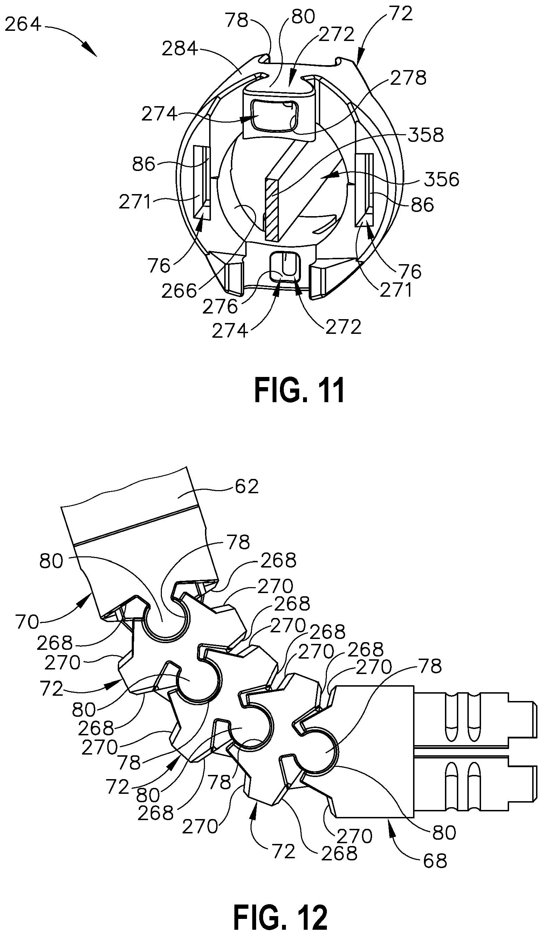

With respect to FIGS. 11-12, slots (86) in each link (68, 70, 72) that collectively define channels (76) are configured to slidably receive articulation bands (74) (*see* FIG. 10). Slots (86) also have drafted openings (271) to inhibit kinking of articulation bands *(74) (see* FIG. 10) during use. Additional control members (not shown), such as additional drivers (not shown), are also connected between end effector (16) (*see* FIG. 7) and base assembly (212) (*see* FIG. 7) and thus extend through distal articulation section (264) in the present example. These additional control members (not shown) are received through arcuate tongues and grooves (80, 78) along lateral centerline to inhibit changing lengths associated with articulation of distal articulation section (264).

More particularly, a pair of passageways (272) longitudinally extend through each link (68, 70, 72) in alignment with arcuate tongues and grooves (80, 78) to collectively define a pair of additional channels (274) configured to guide control members (not shown) through distal articulation section (264). Each passageway (272) also has a widened groove opening (276) and a widened tongue opening (278) as respectively applicable to arcuate groove and tongues (78, 80) of links (68, 70, 72). Each of widened groove and tongue opening (276, 278) is drafted to inhibit kinking of additional control members (not shown) while articulating distal articulation section (264) as described herein. In one example, links (68, 70, 72) may further include a material sleeve (not shown) or material coating (not shown) configured to further inhibit kinking and/or inhibit damage to flexible portion (358) of acoustic waveguide (356) in case of incidental contact.

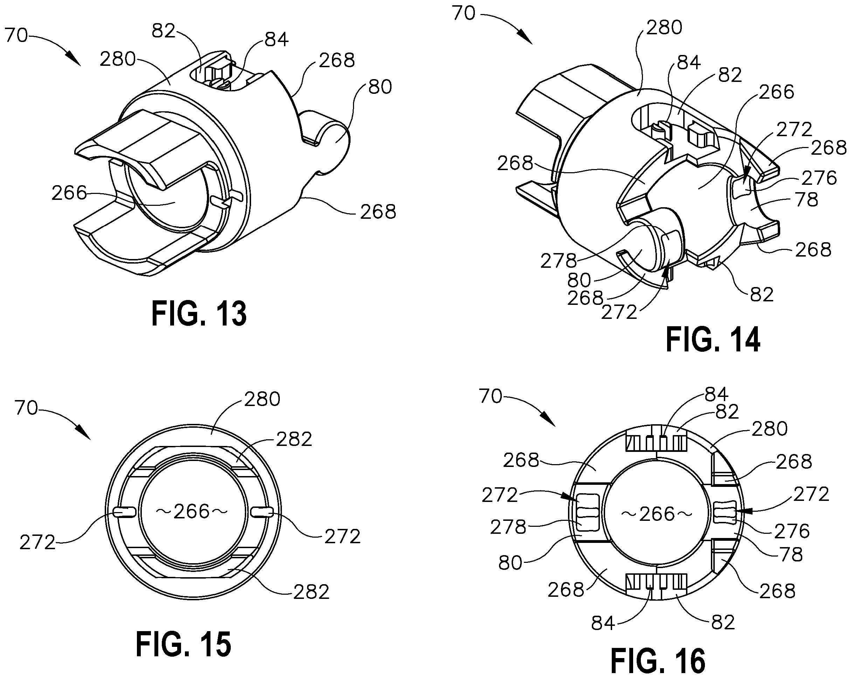

FIGS. 13-16 show distal link (70) in greater detail in one example having a distal link body (280) with proximally extending arcuate tongue (80) and proximally extending arcuate groove (78) with passageways (272). Distal link body (280) further includes distally extending coupling members (282) configured to be received within another portion of shaft assembly (214) (*see* FIG. 7) for rigidly connecting therewith. Notches (82) and pins (84) configured to connect to articulation bands (74) (*see* FIG. 10) are also shown angularly between arcuate tongue and groove (80, 78), whereas distal stops (268) are respectively positioned about arcuate tongue and groove (80, 78). Of course, distal link (70) may vary as desired for incorporating distal articulation section (264) (*see* FIG. 7) into shaft assembly (214) (*see* FIG. 7) such that the disclosure is not intended to be unnecessarily limited to the particular distal link (70) shown in the present example.

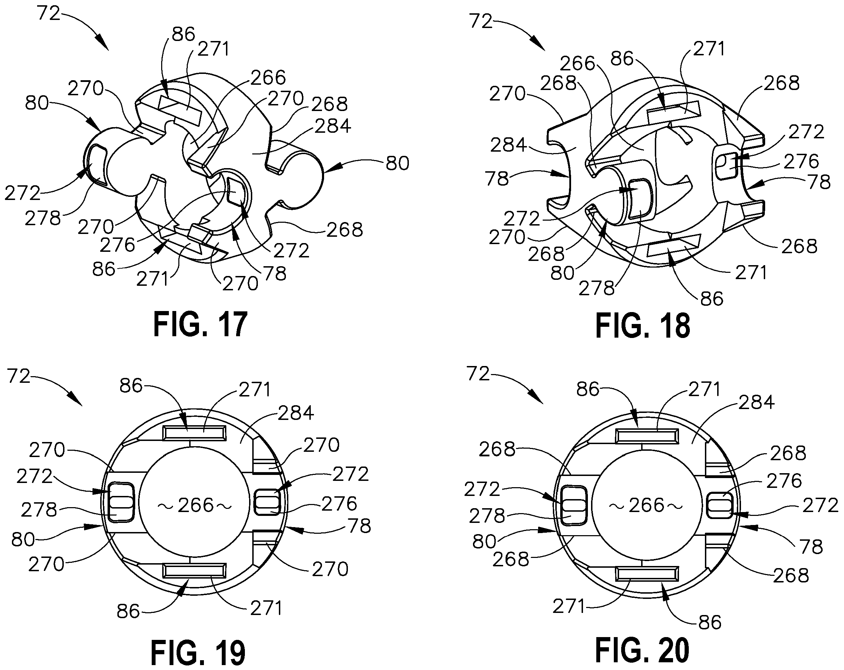

FIGS. 17-20 show intermediate link (72) in greater detail in one example having an intermediate link body (284) with proximally and distally extending arcuate tongues (80) and proximally and distally extending arcuate grooves (78) with passageways (272). Distal stops (268) are respectively positioned about distally facing arcuate tongue and groove (80, 78), whereas proximal stops (270) are respectively positioned about proximally facing arcuate tongue and groove (80, 78). Of course, intermediate link (72) may vary as desired for incorporating distal articulation section (264) (*see* FIG. 7) into shaft assembly (214) (*see* FIG. 7) such that the disclosure is not intended to be unnecessarily limited to the particular intermediate link (72) shown in the present example.

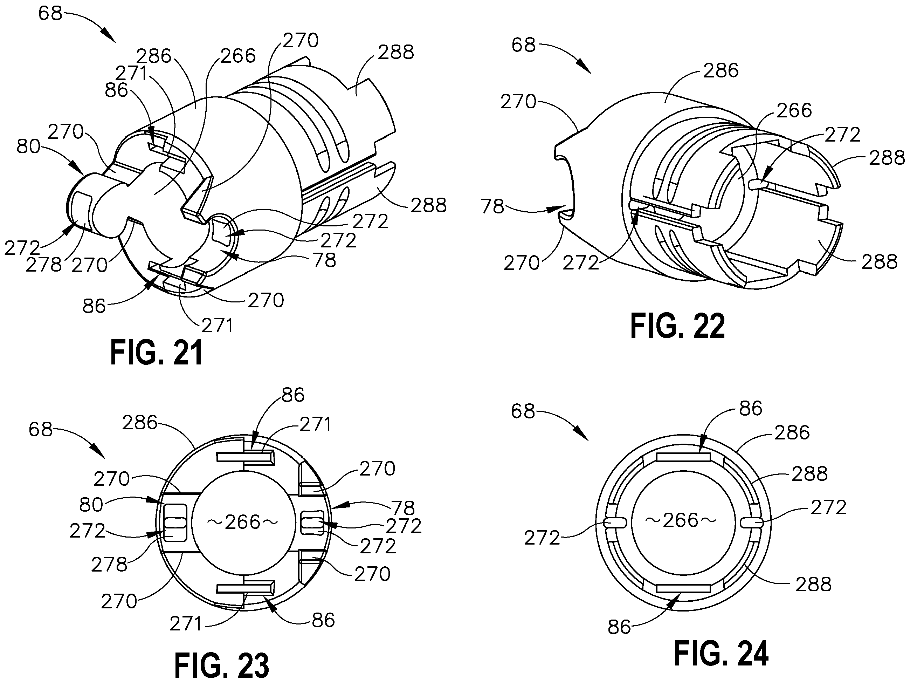

FIGS. 21-24 show proximal link (68) in greater detail in one example having a proximal link body (286) with distally extending arcuate tongue (80) and distally extending arcuate groove (78) with passageways (272). Proximal link body (286) further includes proximally extending coupling members (288) configured to be received within another portion of shaft assembly (214) (*see* FIG. 7) for rigidly connecting therewith. Slots (86) are configured to receive articulation bands (74) (*see* FIG. 10) and shown angularly between arcuate tongue and groove (80, 78), whereas proximal stops (270) are respectively positioned about arcuate tongue and groove (80, 78). Of course, proximal link (68) may vary as desired for incorporating distal articulation section (264) (*see* FIG. 7) into shaft assembly (214) (*see* FIG. 7) such that the disclosure is not intended to be unnecessarily limited to the particular proximal link (68) shown in the present example.

In use, referring back to FIGS. 7-8, the operator selectively directs proximal and distal articulation sections (64, 264) in order to deflect end effector (16) relative to longitudinal axis (61). In one example, proximal articulation section (64) articulates in order to deflect a distal remainder of shaft assembly (214) with end effector (16) through the pitch plane relative to axis (61) and then distal articulation section (264) articulates in order to deflect a further distal remainder of shaft assembly (214) with end effector (16) through the yaw plane relative to axis (374). In another example, distal articulation section (264) articulates in order to deflect the further distal remainder of shaft assembly (214) with end effector (16) through the yaw plane and then proximal articulation section (64) articulates in order to deflect a distal remainder of shaft assembly (214) with end effector (16) through the pitch plane. In still another example, proximal and distal articulation sections (64, 264) simultaneously articulate in order to deflect remainders of shaft assembly (14) and end effector through the pitch and yaw planes respectively. Alternatively, either one of proximal or distal articulation sections (64, 264) are articulated without articulating the remaining of the proximal or distal articulation sections (64, 264). In any case, end effector (16) is thereby configured to deflect through at least two distinct planes via one or more articulation sections (64, 264).

While the present example provides two distinct planes through which to move end effector (16) via two respective articulation sections (64, 264), an alternative articulation section may be configured to provide articulation in at least two distinct planes in a series of joints at discrete longitudinal positions, similar to shaft assembly (214) with articulation sections (64, 264), or in a single joint capable of articulating through at least two planes in one discrete longitudinal position. The disclosure is thus not intended to be unnecessarily limited to multiple articulation sections as shown in the present example for multi-planar articulation as will be appreciated in view of various multi-flex acoustic waveguides (356, 456, 556, 656, 756, 856) discussed below in greater detail.

B. Exemplary Acoustic Waveguides with Flexible Portions for Multi-Planar Articulation

While movement of end effector (16) in six degrees of freedom may increase access to an anatomy of the patient during a surgical procedure for improved patient outcomes, such flexibility tends to strain components, particularly those components configured to communicate ultrasonic vibrations from transducer assembly (54) to ultrasonic blade (46). By way of example, acoustic waveguide (56) of ultrasonic surgical instrument (10) in FIG. 4A is configured to flex at one such flexible portion (58) within articulation section (64) through one plane, but further flexing through another plane would overly strain acoustic waveguide (56) resulting in damage and ultimately failure of acoustic waveguide (56). Such damage and failure of acoustic waveguide (56) tends to occur, because forced flexing of acoustic waveguide (56) generates stress concentrations in one or more locations along acoustic waveguide (56). In turn, these locations of stress concentrations within acoustic waveguide (56) continue to carry the ultrasonic vibrations, resulting in damage, fracture, and failure of acoustic waveguide (56) in use.

Multi-flex acoustic waveguides (356, 456, 556, 656, 756, 856) discussed below with respect to FIGS. 25-35B are thus configured to provide flexing in more than one plane with increased durability. More particularly, multi-flex acoustic waveguides (356, 456, 556, 656, 756, 856) have one or more structural formations configured to communicate ultrasonic vibrations while being flexed in one or more of a variety of available planes of deflection. While shaft assembly (214) (*see* FIG. 8) discussed above incorporates acoustic waveguide (356), it will be appreciated that any other waveguides (456, 556, 656, 756, 856) may also be incorporated into shaft assembly (214), such that the disclosure is not intended to be unnecessarily limited to use with shaft assembly (214) (*see* FIG. 8) discussed above. Like numbers below indicate like features described above in greater detail.

i. A First Exemplary Multi-flex Acoustic Waveguide

FIGS. 25-26 show the first exemplary multi-flex acoustic waveguide (356) having proximal flexible portion (58) and distal flexible portion (358) configured to respectively flex in a pitch direction through the pitch plane and in a yaw direction through the yaw plane. Acoustic waveguide (356) of the present example more particularly includes a proximal waveguide body portion (360) defining a longitudinal axis (361), a distal waveguide body portion (362) distally extending to an ultrasonic blade (346), and an articulation body portion (364) longitudinally extending therebetween. Articulation body portion (364) having proximal and distal flexible portions (58, 358) is thus configured to flex in the pitch and yaw directions to thereby deflect ultrasonic blade (346) relative to longitudinal axis (361) through the pitch and yaw planes for multi-planar deflection. In the present example of proximal waveguide body portion (360), articulation body portion (364), distal waveguide body portion (362), and ultrasonic blade (346) have a single, unitary construction, although multi-flex acoustic waveguide (356) may be alternatively constructed with one or more connected structures. The disclosure is thus not intended to be unnecessarily limited to single, unitary construction of multi-flex acoustic waveguide (356) shown in the present example.

More particularly, proximal flexible portion (58) includes a flexible proximal pitch ribbon (366), whereas distal flexible portion (358) includes a flexible distal yaw ribbon (368). Articulation body portion (364) also includes an intermediate waveguide body portion (370), extending between and in direct connection with flexible proximal pitch ribbon (366) and flexible distal yaw ribbon (368). Bosses (372) are positioned on waveguide body portions (360, 362, 370) and respectively spaced apart from each other so as to coincide with and, more particularly, be centered on respective acoustic nodes along multi-flex acoustic waveguide (356). Similarly, flexible proximal pitch ribbon (366) is positioned and centered on an acoustic antinode of multi-flex acoustic waveguide (356), while flexible distal yaw ribbon (368) is also positioned and centered on another acoustic antinode of multi-flex acoustic waveguide (356).

FIGS. 25-26 show multi-flex acoustic waveguide (356) with each of flexible proximal pitch ribbon (366) and flexible distal yaw ribbon (368) in a linear form such that multi-flex acoustic waveguide (356) has a straight contour. Each ribbon (366, 368) is thereby configured to communicate ultrasonic vibrations in the straight contour longitudinally toward ultrasonic blade (346) during use. Selectively bending flexible proximal pitch ribbon (366) relative to longitudinal axis (361) away from longitudinal axis (361) results in one of any available arcuate contours for flexible proximal pitch ribbon (366). Alternatively or in addition, selectively bending flexible distal yaw ribbon (368) relative an intermediate axis (374) defined by intermediate waveguide body portion (370) away from intermediate axis (374) results in one of any available arcuate contours for flexible distal yaw ribbon (368). In one example, articulation body portion (364) extends along the arcuate contour of only one of flexible proximal pitch ribbon (366) or flexible distal yaw ribbon (368) such that ultrasonic blade (346) deflects through one of two available planes along a blade axis (376). In another example, articulation body portion (364) extends along the arcuate contour of both flexible proximal pitch ribbon (366) and flexible distal yaw ribbon (368) for a dual arcuate contour such that ultrasonic blade (346) deflects through each of two available planes along blade axis (376). While the present example has ribbons (366, 368) angularly oriented perpendicular to each to each other, one or both ribbons (366, 368) may have any relative angular orientation and are not intended to be limited to the angular orientation as shown and described herein.

ii. A Second Exemplary Multi-flex Acoustic Waveguide

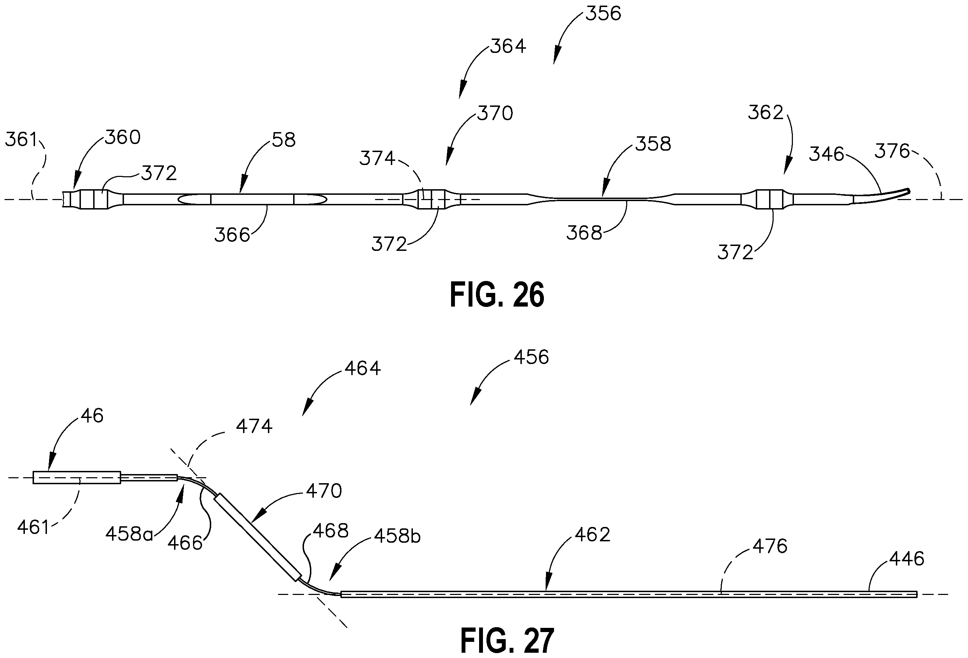

FIG. 27 shows a second exemplary multi-flex acoustic waveguide (456) having a proximal flexible portion (458a) and a distal flexible portion (458b) configured to respectively flex in a yaw direction through a proximal yaw plane and again in a yaw direction through a distal yaw plane. Acoustic waveguide (456) of the present example more particularly includes a proximal waveguide body portion (460) defining a longitudinal axis (461), a distal waveguide body portion (462) distally extending to an ultrasonic blade (446), and an articulation body portion (464) longitudinally extending therebetween. Articulation body portion (464) having proximal and distal flexible portions (458a, 458b) is thus configured to flex in the yaw direction at multiple locations to thereby deflect ultrasonic blade (446) relative to longitudinal axis (461) through multiple yaw planes for multi-planar deflection. In the present example proximal waveguide body portion (460), articulation body portion (464), distal waveguide body portion (462), and ultrasonic blade (446) have a single, unitary construction, although multi-flex acoustic waveguide (456) may be alternatively constructed with one or more connected structures. The disclosure is thus not intended to be unnecessarily limited to single, unitary construction of multi-flex acoustic waveguide (456) shown in the present example.

More particularly, proximal flexible portion (458a) includes a flexible proximal yaw ribbon (466), whereas distal flexible portion (458b) includes a flexible distal yaw ribbon (468). Articulation body portion (464) also includes an intermediate waveguide body portion (470), extending between and in direct connection with flexible proximal yaw ribbon (466) and flexible distal yaw ribbon (468). Bosses (not shown) may be positioned on waveguide body portions (360, 362, 370) and respectively spaced apart from each other so as to coincide with and, more particularly, be centered on respective acoustic nodes along multi-flex acoustic waveguide (456). Similarly, flexible proximal yaw ribbon (466) is positioned and centered on an acoustic antinode of multi-flex acoustic waveguide (456), while flexible distal yaw ribbon (468) is also positioned and centered on another acoustic antinode of multi-flex acoustic waveguide (456).

Each ribbon (436, 468) is configured to communicate ultrasonic vibrations in a straight contour longitudinally toward ultrasonic blade (446) during use. Selectively bending flexible proximal yaw ribbon (466) relative to longitudinal axis (461) away from longitudinal axis (461) results in one of any available arcuate contours for flexible proximal yaw ribbon (466). Alternatively or in addition, selectively bending flexible distal yaw ribbon (468) relative an intermediate axis (474) defined by intermediate waveguide body portion (470) away from intermediate axis (474) results in one of any available arcuate contours for flexible distal yaw ribbon (468). In one example, articulation body portion (464) extends along the arcuate contour of only one of flexible proximal yaw ribbon (466) or flexible distal yaw ribbon (468) such that ultrasonic blade (446) deflects through one of two available planes along a blade axis (476). In another example, articulation body portion (464) extends along the arcuate contour of both flexible proximal yaw ribbon (466) and flexible distal yaw ribbon (468) for a dual arcuate contour such that ultrasonic blade (446) deflects through each of two available planes along blade axis (476) and as shown in FIG. 27. While the present example has ribbons (466, 468) in the same angular orientation, one or both ribbons (466, 468) may have any relative angular orientation and are not intended to be limited to the angular orientation as shown and described herein.

iii. A Third Exemplary Multi-flex Acoustic Waveguide

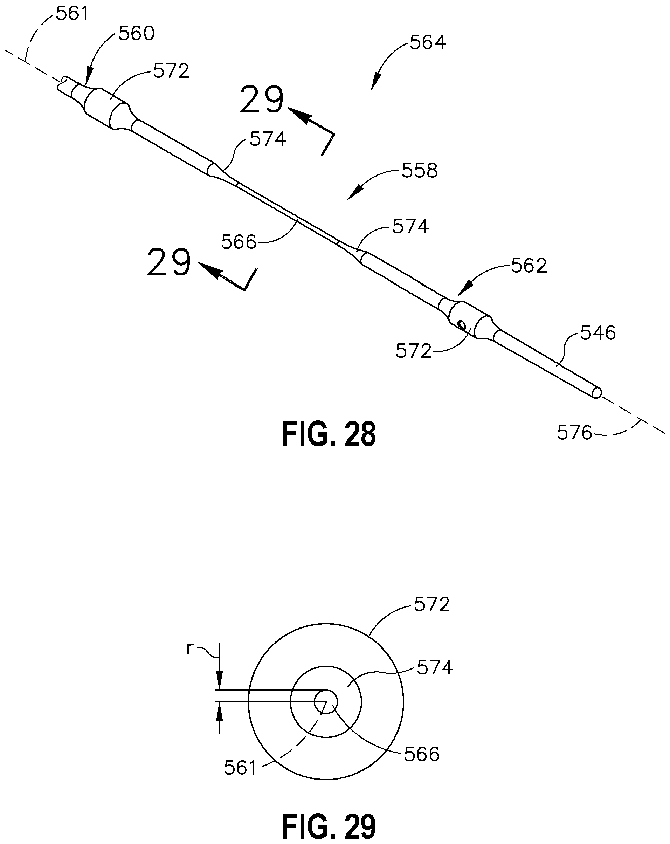

FIGS. 28-30B show a third exemplary multi-flex acoustic waveguide (556) having a flexible portion (558) configured to flex in a full 360-degree range of radial directions through a respective full 360-degree range of radial planes. Acoustic waveguide (556) of the present example more particularly includes a proximal waveguide body portion (560) defining a longitudinal axis (561), a distal waveguide body portion (562) distally extending to an ultrasonic blade (546), and an articulation body portion (564) longitudinally extending therebetween. Articulation body portion (564) having flexible portion (558) is thus configured to flex in any radial direction about longitudinal axis (561) to thereby deflect ultrasonic blade (546) relative to longitudinal axis (561) through any respective radial plane for multi-planar deflection. In the present example, proximal waveguide body portion (560), articulation body portion (564), distal waveguide body portion (562), and ultrasonic blade (546) have a single, unitary construction, although multi-flex acoustic waveguide (556) may be alternatively constructed with one or more connected structures. The disclosure is thus not intended to be unnecessarily limited to single, unitary construction of multi-flex acoustic waveguide (556) shown in the present example.

More particularly, as shown in FIGS. 28-29, flexible portion (558) includes a first example of a flexible wire (566) configured to flex in any radial direction about longitudinal axis (561) to thereby deflect ultrasonic blade (546) relative to longitudinal axis (561) through any respective radial plane for multi-planar deflection. Flexible wire (566) is elongated and cylindrical defining a wire cross-sectional radius (r). Bosses (572) are positioned on waveguide body portions (560, 562) and are respectively spaced apart from each other so as to coincide with and, more particularly, be centered on respective acoustic nodes along multi-flex acoustic waveguide (556). Similarly, flexible wire (566) is positioned and centered on an acoustic antinode of multi-flex acoustic waveguide (556). Each proximal and distal waveguide body portion (560, 562) is more rigid than flexible wire (566) and has a conical taper (574) narrowing toward flexible wire (566). Between conical tapers (574) and bosses (572), proximal and distal waveguide body portions (560, 562) each define a waveguide body radius. In the present example, waveguide body radius is larger than wire cross-sectional radius (r).

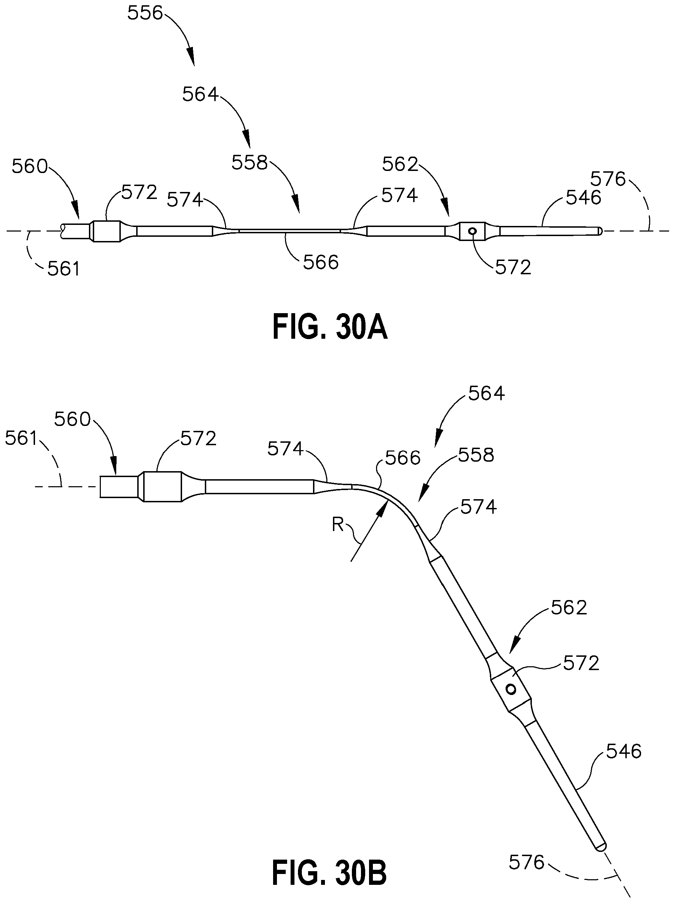

FIGS. 30A shows multi-flex acoustic waveguide (556) with flexible wire (566) in a linear form such that multi-flex acoustic waveguide (556) has a straight contour. Flexible wire (566) is thereby configured to communicate ultrasonic vibrations in the straight contour longitudinally toward ultrasonic blade (546) during use. Selectively bending flexible wire (566) relative to longitudinal axis (561) away from longitudinal axis (561) results in one of any available arcuate contours for flexible wire (566) with ultrasonic blade (346) deflecting through one of any available radial plane along a blade axis (376) and about a bend radius (R). One example of such bend radius (R) is shown in FIG. 30B. With the arcuate contour, flexible wire (566) is configured to uncouple a longitudinal vibrational component of the ultrasonic vibration from a transverse vibrational component of the ultrasonic vibration to thereby communicate the ultrasonic vibration about the bent flexible wire (566) without damaging flexible wire (566) or substantially degrading the ultrasonic vibration during use.

To this end, acoustic waveguide (556) has a set of predetermined properties to uncouple the longitudinal vibrational component of the ultrasonic vibration from the transverse vibrational component of the ultrasonic vibration during use. In the present example, the predetermined properties include wire cross-sectional radius (r) and bend radius (R) discussed above in addition to a plurality of wire material properties, including an elastic modulus (E) of flexible wire (566), a yield strength ($\sigma_y$) of flexible wire (566), a natural frequency (f) of flexible wire (566), as well as a constant, the speed of sound (c). Given that bend radius (R) may vary, predetermined properties further include a first condition, a second condition, and a third condition that accommodate a range of available bend radii (R) while still effectively uncoupling the longitudinal vibrational component of the ultrasonic vibration from the transverse vibrational component of the ultrasonic vibration during use. As shown in one example, these conditions are as follows.

$$\text{First Condition:} \quad \frac{r}{R} < 0.1$$

$$\text{Second Condition:} \quad R > \frac{c}{2\pi f}$$

$$\text{Third Condition:} \quad \frac{8Er}{\pi^2 R} < \sigma_y$$

While the particular material, sizing, and bend of flexible wire (566) may vary to achieve uncoupling of the longitudinal vibrational component of the ultrasonic vibration from the transverse vibrational component of the ultrasonic vibration, in one example the material is a nitinol material. In another example, the material of flexible wire (566) is a titanium material. The invention is thus not intended to be unnecessarily limited to the particular material, sizing, and bend of flexible wire (566) as shown and described herein.

With respect to FIG. 30B, flexible wire (566) has one bend about an acoustic antinode with bend radius (R). Alternatively or in addition, an alternative flexible wire (not shown) may have an additional bend (not shown) about another antinode such that flexible wire (not shown) is configured to bend at two or more positions during use, similar to dual flexible portions (58, 358, 458a, 458b) (*see* FIGS. 25-27) associated with acoustic waveguides (356, 456) (*see* FIGS. 25-27) discussed above in greater detail. Such an alternative flexible wire (not shown) may be offset from flexible wire (566) with an intermediate waveguide body portion (not shown) therebetween similar to acoustic waveguides (356, 456) (*see* FIGS. 25-27) or without intermediate waveguide body portion (not shown) such that alternative flexible wire (not shown) and flexible wire (566) are essentially continuous therealong. The disclosure is thus not intended to be unnecessarily limited to the arrangement of one flexible wire (566) as shown in the present example.

iv. A Fourth Exemplary Multi-flex Acoustic Waveguide

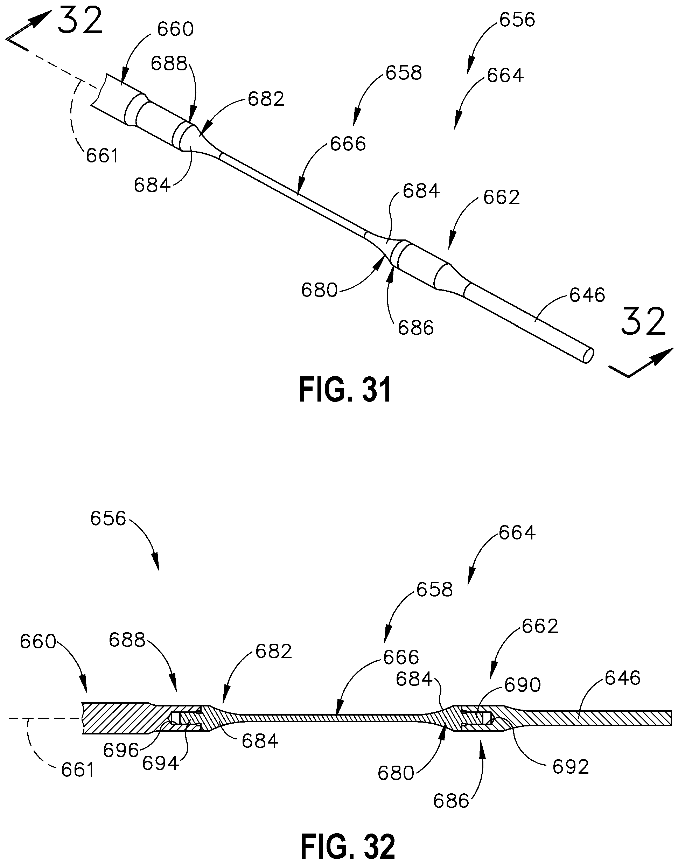

FIGS. 31-32 show a fourth exemplary multi-flex acoustic waveguide (656) having a flexible portion (658) configured to flex in a full 360-degree range of radial directions through a respective full 360-degree range of radial planes. Acoustic waveguide (656) of the present example more particularly includes a proximal waveguide body portion (660) defining a longitudinal axis (661), a distal waveguide body portion (662) distally extending to an ultrasonic blade (646), and an articulation body portion (664) longitudinally extending therebetween. Articulation body portion (664) having flexible portion (658) is thus configured to flex in any radial direction about longitudinal axis (661) to thereby deflect ultrasonic blade (646) relative to longitudinal axis (661) through any respective radial plane for multi-planar deflection.

More particularly, flexible portion (658) includes a second example of a flexible wire (666) configured to flex in any radial direction about longitudinal axis (661) to thereby deflect ultrasonic blade (646) relative to longitudinal axis (661) through any respective radial plane for multi-planar deflection. In this respect, acoustic waveguide (656) is similar to acoustic waveguide (556) (*see* FIG. 28), but, rather than being unitarily constructed, acoustic waveguide (656) is assembled via several discrete components. Flexible wire (666) is like flexible wire (566) (*see* FIG. 28) discussed above in other respects unless otherwise stated below.

As shown in the present example, flexible portion (658) further includes a distal wire end portion (680) opposite from a proximal wire end portion (682) with flexible wire (666) extending therebetween. Distal and proximal wire end portions (680, 682) each have a conical taper (684) narrowing toward flexible wire (666). Extending opposite from respective conical tapers (684), distal end proximal wire end portions (680, 682) further respectively include a distal coupling (686) configured to connect to distal waveguide body portion (662) and a proximal coupling (688) configured to connect to proximal waveguide body portion (660). As shown in the present example, distal coupling (686) includes a distal threaded stud (690) distally extending from distal wire end portion (680) and a distal threaded bore (692) in distal waveguide body portion (662). Distal threaded stud (690) mechanically and acoustically couples into distal threaded bore (692) to connect flexible wire (666) to distal waveguide body portion (662). Similarly, proximal coupling (688) includes a proximal threaded stud (694) proximally extending from proximal wire end portion (682) and a proximal threaded bore (696) in proximal waveguide body portion (660). Proximal threaded stud (694) mechanically and acoustically couples into proximal threaded bore (696) to connect flexible wire (666) to proximal waveguide body portion (660).

With acoustic waveguide (656) assembled via several discrete components, one or more of proximal waveguide body potion (660), distal waveguide body portion (662), and articulation body portion (664) may be formed from differing materials. By way of example, proximal waveguide body potion (660) is formed from one of titanium material, aluminum, material, or nitinol material. In addition, distal waveguide body potion (662), which includes ultrasonic blade (646), is formed from one of titanium material or nitinol material. Similarly, articulation body portion (664) is formed from one of titanium material or nitinol material. Any combination of such materials may be incorporated into acoustic waveguide (656) and thus configured to uncouple the longitudinal vibrational component of the ultrasonic vibration from the transverse vibrational component of the ultrasonic vibration based on the set of predetermined properties discussed above in greater detail. While the present example incorporates threads into acoustic waveguide (656) for connecting various components of differing materials, such connections may additionally or alternatively include swaging, welding, temperature fits, and/or shape memory fits. The disclosure is thus not intended to be unnecessarily limited to the particular threaded couplings (686, 688) shown and described in the present example.

v. A Fifth Exemplary Multi-flex Acoustic Waveguide

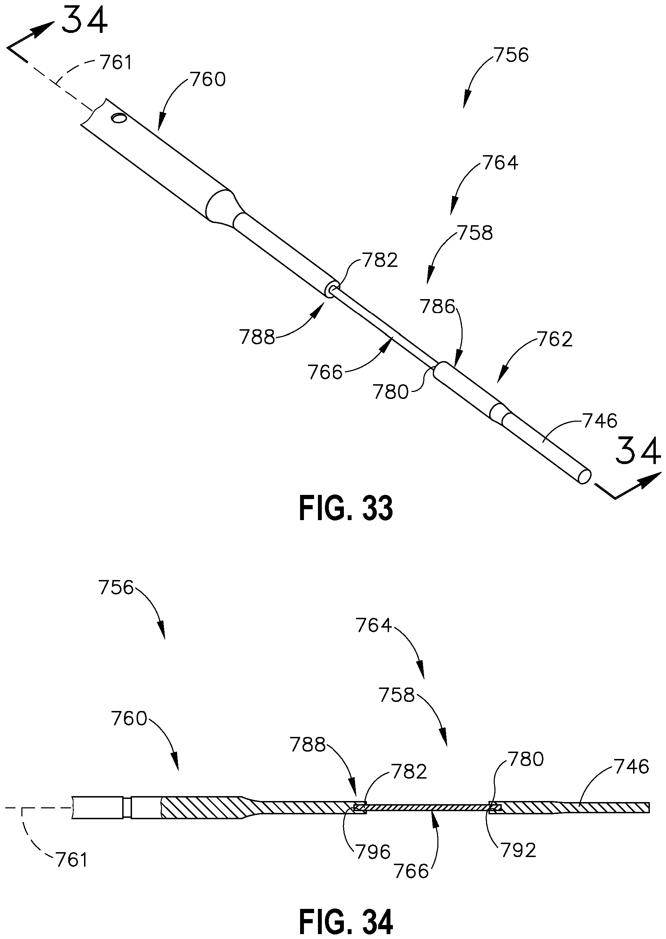

FIGS. 33-34 show a fifth exemplary multi-flex acoustic waveguide (756) having a flexible portion (758) configured to flex in a full 360-degree range of radial directions through a respective full 360 degree range of radial planes. Acoustic waveguide (756) of the present example more particularly includes a proximal waveguide body portion (760) defining a longitudinal axis (761), a distal waveguide body portion (762) distally extending to an ultrasonic blade (746), and an articulation body portion (764) longitudinally extending therebetween. Articulation body portion (764) having flexible portion (758) is thus configured to flex in any radial direction about longitudinal axis (761) to thereby deflect ultrasonic blade (746) relative to longitudinal axis (761) through any respective radial plane for multi-planar deflection.

More particularly, flexible portion (758) includes a third example of a flexible wire (766) configured to flex in any radial direction about longitudinal axis (761) to thereby deflect ultrasonic blade (746) relative to longitudinal axis (761) through any respective radial plane for multi-planar deflection. In this respect, acoustic waveguide (756) is similar to acoustic waveguide (556) (*see* FIG. 28), but, rather than being unitarily constructed, acoustic waveguide (756) is assembled via several discrete components. Flexible wire (766) is like flexible wire (566) (*see* FIG. 28) discussed above in other respects unless otherwise stated below.

As shown in the present example, flexible portion (758) further includes a distal wire end portion (780) opposite from a proximal wire end portion (782) with flexible wire (766) extending therebetween. Distal and proximal wire end portions (780, 782) respectively include a distal coupling (786) configured to connect to distal waveguide body portion (762) and a proximal coupling (788) configured to connect to proximal waveguide body portion (760). As shown in the present example, distal coupling (786) includes distal wire end portion (780) and a distal bore (792) in distal waveguide body portion (762). Distal wire end portion (780) is swaged into distal bore (792) to thereby mechanically and acoustically couple flexible wire (766) to distal waveguide body portion (762). Similarly, proximal coupling (788) includes proximal wire end portion (682) and a proximal bore (796) in proximal waveguide body portion (760). Proximal wire end portion (782) is swaged into proximal bore (796) to thereby mechanically and acoustically couple flexible wire (766) to proximal waveguide body portion (760).

With acoustic waveguide (756) assembled via several discrete components, one or more of proximal waveguide body potion (760), distal waveguide body portion (762), and articulation body portion (764) may be formed from differing materials. By way of example, proximal waveguide body potion (760) is formed from one of titanium material, aluminum, material, or nitinol material. In addition, distal waveguide body potion (762), which includes ultrasonic blade (746), is formed from one of titanium material or nitinol material. Similarly, articulation body portion (764) is formed from one of titanium material or nitinol material. Any combination of such materials may be incorporated into acoustic waveguide (756) and thus configured to uncouple the longitudinal vibrational component of the ultrasonic vibration from the transverse vibrational component of the ultrasonic vibration based on the set of predetermined properties discussed above in greater detail. While the present example swages portions of acoustic waveguide (656) together for connecting various components of differing materials, such connections may additionally or alternatively include threading, welding, temperature fits, and/or shape memory fits. The disclosure is thus not intended to be unnecessarily limited to the particular swaging shown and described in the present example.

vi. A Sixth Exemplary Multi-flex Acoustic Waveguide

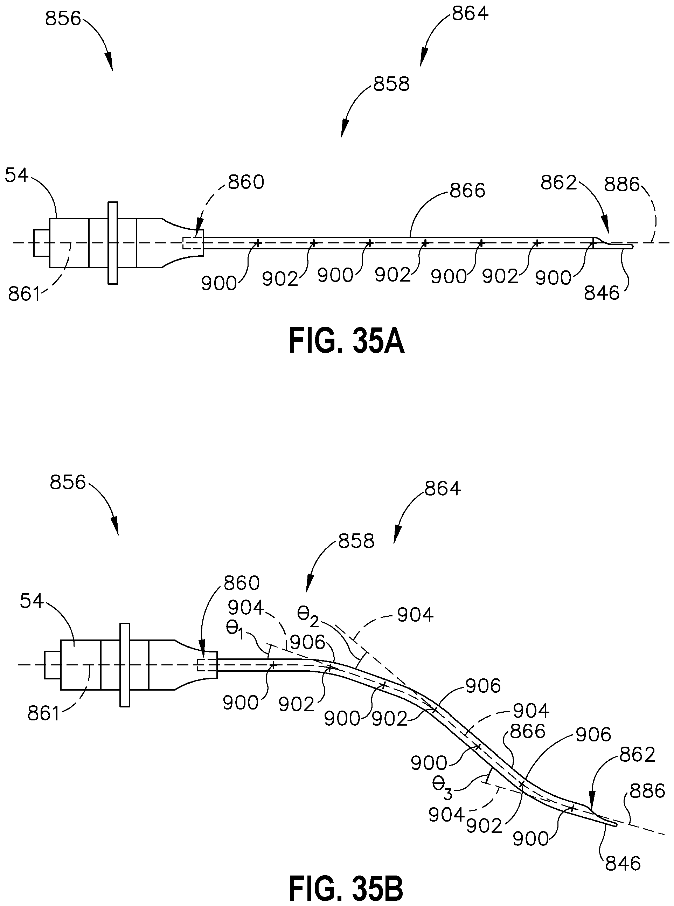

FIGS. 35A-35B show a sixth exemplary multi-flex acoustic waveguide (856) having a flexible portion (858) configured to flex in a full 360-degree range of radial directions through a respective full 360-degree range of radial planes. Acoustic waveguide (856) of the present example more particularly includes a proximal waveguide body portion (860) defining a longitudinal axis (861), a distal waveguide body portion (862) distally extending to an ultrasonic blade (846), and an articulation body portion (864) longitudinally extending therebetween. Articulation body portion (864) having flexible portion (858) is thus configured to flex in any radial direction about longitudinal axis (861) to thereby deflect ultrasonic blade (846) relative to longitudinal axis (861) through any respective radial plane for multi-planar deflection. In the present example, proximal waveguide body portion (860), articulation body portion (864), distal waveguide body portion (862), and ultrasonic blade (846) have a single, unitary construction, although multi-flex acoustic waveguide (856) may be alternatively constructed with one or more connected structures. The disclosure is thus not intended to be unnecessarily limited to single, unitary construction of multi-flex acoustic waveguide (856) shown in the present example.

More particularly, flexible portion (558) includes an elongate flexible wire (866) extending essentially an entire length of articulation body portion (864) such that proximal waveguide body portion (860) is a proximal portion of acoustic waveguide (856) configured to be received within transducer assembly (54) and distal waveguide body portion (862) is generlly ultrasonic blade (846). A majority of acoustic waveguide (856) is thus elongate flexible wire (866), which extends along a plurality of acoustic nodes (900) and a plurality of acoustic antinodes (902). Thus, elongate flexible wire (866) is configured to flex in any radial direction about longitudinal axis (861) as well as further flex in any radial direction about axes (904), which are positioned and aligned at acoustic nodes (902), respectively. Furthermore, elongate flexible wire (858) of the present example includes a plurality of flexible wire portions (906) respectively centered at acoustic antinodes (902) of multi-flex acoustic waveguide (856). Flexible wire portions (906) and intermediate wire portions are thus continuous to define elongate flexible wire (866) in the present example.

FIGS. 35A shows multi-flex acoustic waveguide (856) with elongate flexible wire (866) in a linear form such that multi-flex acoustic waveguide (856) has a straight contour. Flexible wire (866) is thereby configured to communicate ultrasonic vibrations in the straight contour longitudinally toward ultrasonic blade (846) during use. Selectively bending flexible wire (866) at any flexible wire portion (906) relative to axes (861, 904) results in one of any available arcuate contours for elongate flexible wire (866) with ultrasonic blade (846) deflecting through one of any available radial planes along a blade axis (876). With the arcuate contour shown in FIG. 35B, elongate flexible wire (866) is configured to uncouple a longitudinal vibrational component of the ultrasonic vibration from a transverse vibrational component of the ultrasonic vibration to thereby communicate the ultrasonic vibration about the bent flexible wire (866) without damaging flexible wire (866) or substantially degrading the ultrasonic vibration during use. Such vibrational uncoupling is based on the set of predetermined properties discussed above in greater detail.

While the particular material, sizing, and bend of flexible wire (866) may vary to achieve uncoupling of the longitudinal vibrational component of the ultrasonic vibration from the transverse vibrational component of the ultrasonic vibration, in one example the material is a nitinol material. In another example, the material of flexible wire (866) is a titanium material. In any case, the invention is thus not intended to be unnecessarily limited to the particular material, sizing, and bend of flexible wire (866) as shown and described herein.

III. Ultrasonic Blades with Backcutting Edge and Circumferential Sealing

With respect to FIGS. 3A-3B discussed above in greater detail, clamp arm (44) is configured to rotate about blade (46) and also relative to shaft assembly (14) as indicated by arrow (53). In one example, clamp arm (44) selectively rotates in the clockwise or counterclockwise directions around blade (46) such that the operator selectively fixes clamp arm (44) angularly about blade (46) to thereby clamp tissue between blade (46) and clamp arm (44) with increased access to the tissue. While clamping between blade (46) and clamp arm (44), the operator selectively activates blade (46) with ultrasonic vibrations and, in one example, seals the tissue clamped therebetween. As shown in the present example, blade (46) has a blade body (910) longitudinally extending to a hemispherical distal end tip (912). Blade body (910) and hemispherical distal end tip (912) are generally rounded smooth and free of edges such that blade (46) is axisymmetric and has a full, circular circumferential sealing profile angularly about an entirety of longitudinal axis (61). This full, circular circumferential sealing profile represents engagement between rounded smooth surfaces of blade (46) and clamp pad (48) such that tissue sealing may occur around an entirety of blade (46) with clamp pad (48) clamping tissue thereagainst.

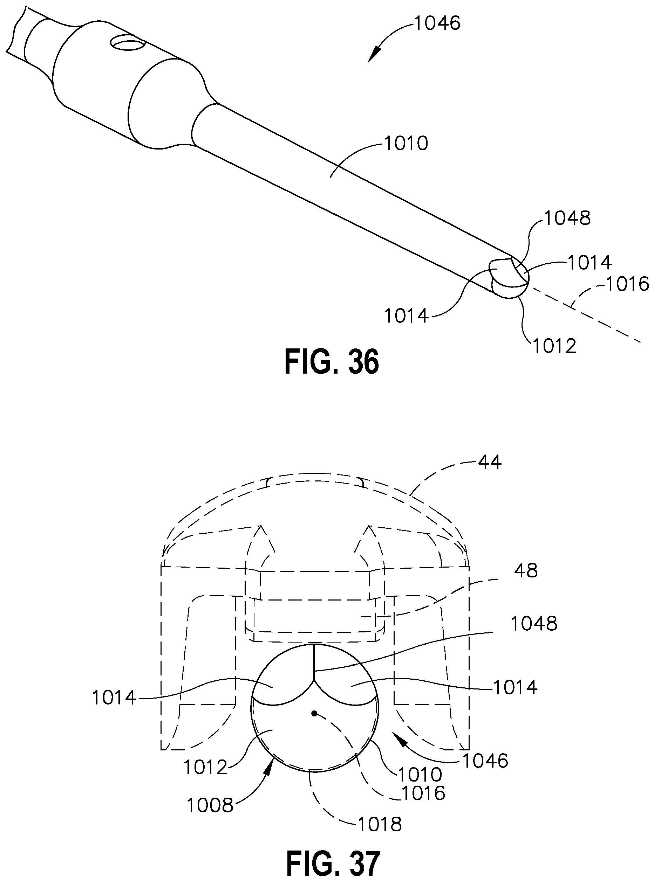

In some instances, it may be desirable to incorporate backcutting functionality into blade (46) while retaining a majority of the circumferential sealing profile about blade (46) for sealing tissue against clamp pad (48). Various examples of ultrasonic blades (1046, 1146) with backcutting edges (1048, 1148) for providing such backcutting functionality to the operator are described below in greater detail with respect to FIGS. 36-39. While backcutting edges (1048, 1148) may be incorporated into blade (46) (*see* FIG. 3A), the disclosure is not intended to be unnecessarily limited to including backcutting functionality. Moreover, it will be appreciated that alternative backcutting edges (not shown) may also have a circumferential sealing profile about a majority of a circumferential blade profile such that the disclosure is also not intended to be limited to the particular backcutting edges (1048, 1148) shown and described herein.

A. First Exemplary Backcutting Edge

FIGS. 36-37 show a first example of a circumferential blade profile of (1008) of an ultrasonic blade (1046) with a first backcutting edge (1048) configured to backcut tissue about a minority of circumferential blade profile (1008) and further configured to seal tissue about a majority of circumferential blade profile (1008). Blade (1046) of the present example more particularly includes a blade body (1010) distally extending to a partially hemispherical distal end tip (1012). A pair of laterally, longitudinally, and transversely swept grooves (1014) extend through partially hemispherical distal end tip (1012) as well as a portion of blade body (1010) to define backcutting edge (1048) along blade (1046). As shown in the present example, a majority of a longitudinal length of backcutting edge (1048) is positioned on partially hemispherical distal end tip (1012) rather than blade body (1010). More particularly, all of the longitudinal length of backcutting edge (1048) is positioned on partially hemispherical distal end tip (1012) in the present example such that no portion of backcutting edge (1048) is positioned on blade body (1010) despite some proximal portion of swept grooves (1014) being on blade body (1010).

As more particularly shown in FIG. 37, backcutting edge (1048) longitudinally extends through a transversely extending plane also in alignment with a central blade axis (1016). Swept grooves (1014) are thus laterally symmetric about this transversely extending plane. Circumferential blade profile (1008) of the present example is circular about an entirety of central blade axis (1016) such that clamp pad (48) extends as a tangent about blade (1046) in any angular position about blade (1046) but for portions of clamp pad (48) adjacent to swept grooves (1014), thereby defining a circumferential sealing profile (1018). Circumferential sealing profile (1018) is thus arcuate and angularly surrounds a majority of central blade axis (1016), such as greater than 180 degrees, without surrounding portions that include swept grooves (1014) and backcutting edge (1048). Circumferential sealing profile (1018) thereby represents the rounded surfaces along partially hemispherical distal end tip (1012) configured for sealing tissue against clamp pad (48). It will be appreciated that backcutting edge (1048) and swept grooves (1014) may vary while still providing circumferential sealing profile (1018) about the majority of central blade axis (1016). The disclosure is thus not intended to be limited to the particular backcutting edge (1048) and swept grooves (1014) shown in the present example.

In use for sealing tissue, the operator selectively rotates clamp arm (44) relative to blade (1046) to position clamp pad (48) in any desirable angular position radially inline with circumferential sealing profile (1018). In turn, tissue is received between clamp pad (48) and blade (1046) against circumferential sealing profile (1018), and clamp arm (44) pivots from the open position to the closed position for clamping tissue against blade (1046). The operator selectively actives blade (1046) with ultrasonic vibrations in order to seal tissue clamped between clamp pad (48) and clamp arm (44). In the event that the operator desires to selectively backcut tissue, clamp arm (44) is positioned away from backcutting edge (1048) such that backcutting edge (1048) is relatively exposed. The operator then directly engages tissue with backcutting edge (1048) for backcutting the tissue as desired.

In one example, clamp arm (44) is further configured to only radially align with the circumferential sealing profile (1018) in order to inhibit the operator from inadvertently moving clamp pad (48) toward direct engagement with backcutting edge (1048). Clamp arm (44) may have mechanical stops (not shown) and/or associated software configured to inhibit such rotation. Of course, the disclosure is not intended to be unnecessarily limited to include such alignment constraints and, in some examples, clamp arm (44) is free to move to any angular position about blade (1046).

B. Second Exemplary Backcutting Edge

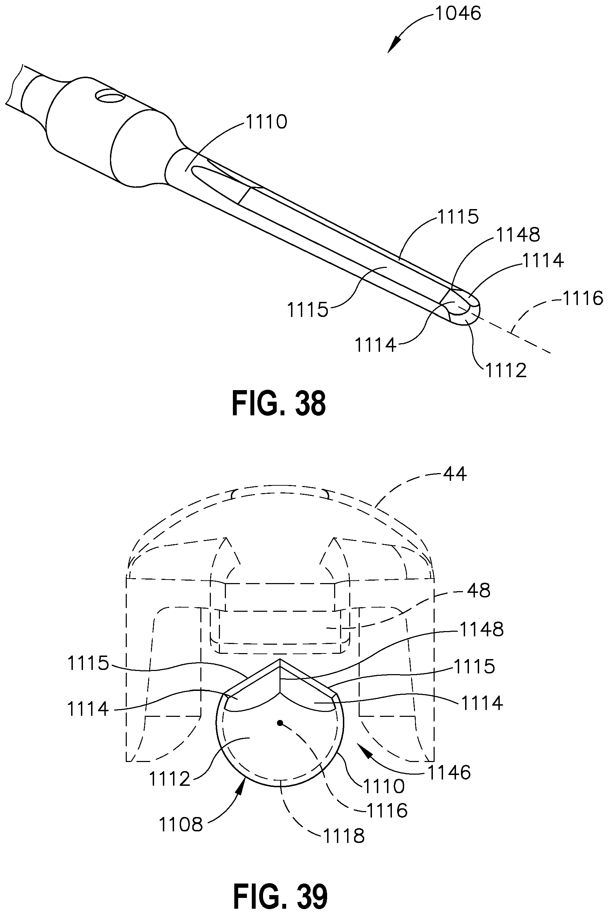

FIGS. 38-39 show a third example of a circumferential blade profile of (1108) of an ultrasonic blade (1146) with a second backcutting edge (1148) configured to backcut tissue about a minority of circumferential blade profile (1108) and further configured to seal tissue about a majority of circumferential blade profile (1108). Blade (1146) of the present example more particularly includes a blade body (1110) distally extending to partially hemispherical distal end tip (1112). A pair of laterally, longitudinally, and transversely swept grooves (1114) extend through partially hemispherical distal end tip (1012). In addition, a pair of laterally, longitudinally, and transversely swept grooves (1115) extend along blade body (1010). Swept grooves (1114) and grooves (1115) collectively define backcutting edge (1148) along blade (1146). As shown in the present example, a majority of a longitudinal length of backcutting edge (1148) is positioned on blade body (1110) such that backcutting edge (1148) extends along majority of a length of blade (1146).

As more particularly shown in FIG. 39, backcutting edge (1148) longitudinally extends through a transversely extending plane also in alignment with a central blade axis (1116). Swept grooves (1114, 1115) are thus laterally symmetric about this transversely extending plane. Circumferential blade profile (1108) of the present example is circular about an angular majority of central blade axis (1016) such that clamp pad (48) extends as a tangent about blade (1046) in this angular majority about blade (1046) but for portions of clamp pad (48) adjacent to swept grooves (1114, 1115), thereby defining a circumferential sealing profile (1118). Circumferential sealing profile (1118) is thus arcuate and angularly surrounds a majority of central blade axis (1116), such as greater than 180 degrees, without surrounding portions that include swept grooves (1114, 1115) and backcutting edge (1148). Circumferential sealing profile (1118) thereby represents the rounded surfaces along partially hemispherical distal end tip (1112) configured for sealing tissue against clamp pad (48). It will be appreciated that backcutting edge (1148) and swept grooves (1114, 1115) may vary while still providing circumferential sealing profile (1118) about the majority of central blade axis (1116). The disclosure is thus not intended to be limited to the particular backcutting edge (1148) and swept grooves (1114, 1115) shown in the present example.

In use for sealing tissue, the operator selectively rotates clamp arm (44) relative to blade (1146) to position clamp pad (48) in any desirable angular position radially inline with circumferential sealing profile (1118). In turn, tissue is received between clamp pad (48) and blade (1146) against circumferential sealing profile (1118), and clamp arm (44) pivots from the open position to the closed position for clamping tissue against blade (1146). The operator selectively actives blade (1146) with ultrasonic vibrations in order to seal tissue clamped between clamp pad (48) and clamp arm (44). In the event that the operator desires to selectively backcut tissue, clamp arm (44) is positioned away from backcutting edge (1148) such that backcutting edge (1148) is relatively exposed. The operator then directly engages tissue with backcutting edge (1148) for backcutting the tissue as desired.

In one example, clamp arm (44) is further configured to only radially align with the circumferential sealing profile (1118) in order to inhibit the operator from inadvertently moving clamp pad (48) toward direct engagement with backcutting edge (1148). Clamp arm (44) may have mechanical stops (not shown) and/or associated software configured to inhibit such rotation. Of course, the disclosure is not intended to be unnecessarily limited to include such alignment constraints and, in some examples, clamp arm (44) is free to move to any angular position about blade (1146).

IV. Exemplary Shift of Acoustic Drivetrain with Shaft Assembly Articulation

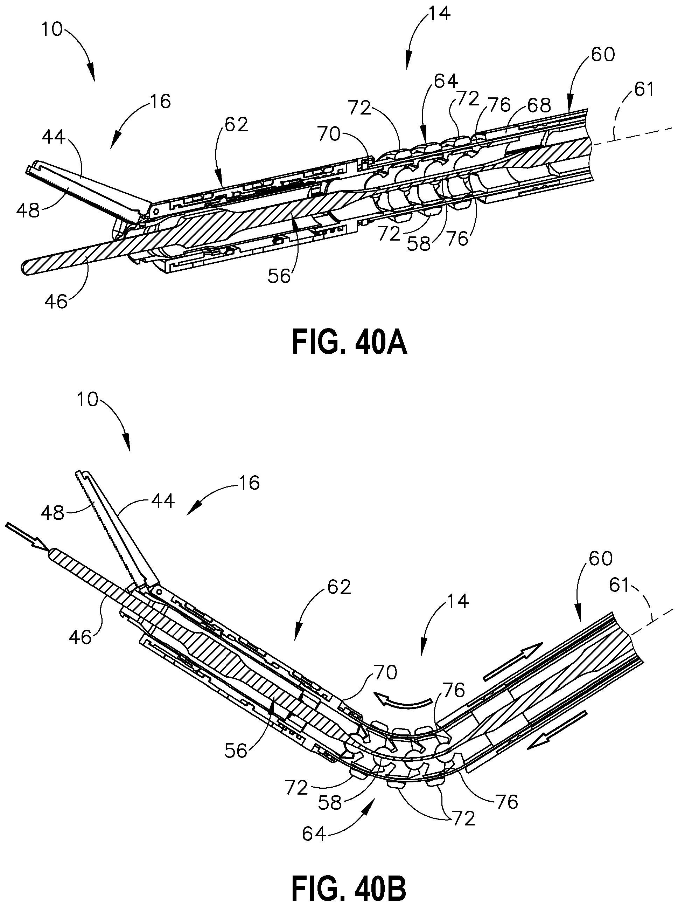

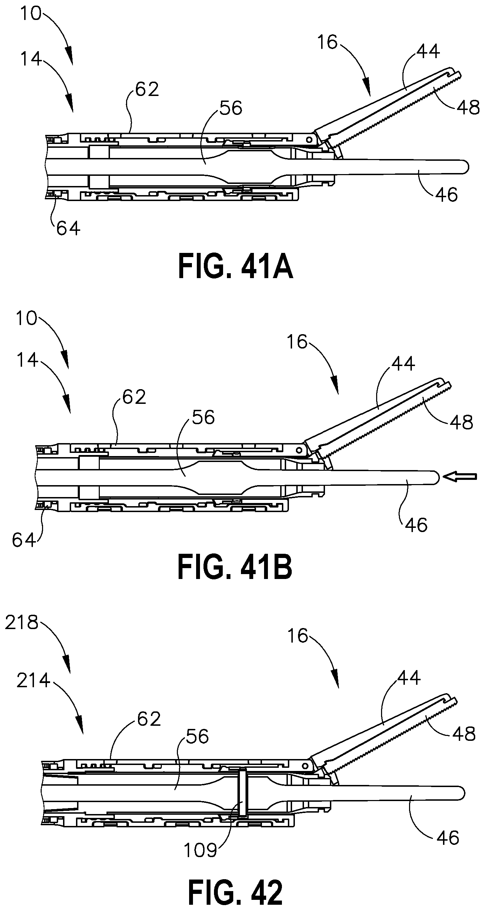

With respect to FIGS. 40A-41B, in one example, the distal tip of blade (46) is positioned to align in a predetermined alignment with a distal tip of clamp arm (44) in the straight configuration as shown in FIG. 40A and FIG. 41A. More particularly, such predetermined alignment positions the distal tip of blade (46) longitudinally flush with distal tip of clamp arm (44) in the closed position so that the distal tips of blade (46) and clamp arm (44) are positioned in a common plane perpendicular to an axis defined by blade (46). As articulation section (64) articulates from the straight configuration toward the articulated configuration as shown in FIG. 40B and FIG. 41B, articulation section (64) essentially elongates as the radius of curvature along articulation section (64) increases. In turn, blade (46) moves proximally relative to clamp arm (44) such that the distal tip of blade (46) and the distal tip of clamp arm (44) are no longer longitudinally aligned in the predetermined alignment due to a constant longitudinal length of acoustic waveguide (56) and blade (46) from transducer assembly (54) (*see* FIG. 6) to the distal tip of blade (46).

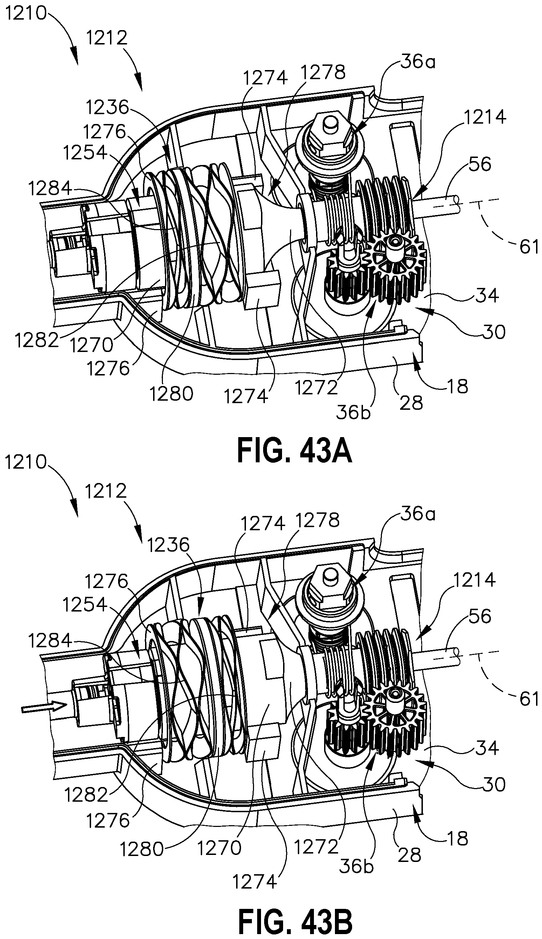

In some instances, it may be desirable to longitudinally adjust blade (46) relative to clamp arm (44) so as to maintain the predetermined alignment between blade (46) and clamp arm (44) with articulation section (64) in the straight and articulated configurations. Given the constant longitudinal length of acoustic waveguide (56) and blade (46), a proximal portion of the acoustic drivetrain, such as transducer assembly (54) (*see* FIG. 5), may be shifted in order to offset shift at a distal portion of the acoustic drivetrain, such as blade (46). To this end, a shiftable transducer assembly (1254, 1354) (*see* FIGS. 43A-44B) may be incorporated into ultrasonic surgical instrument (10) to align blade (46) with clamp arm (44) as desired, such as shown in FIG. 41A. In an alternative example shown in FIG. 42, an ultrasonic surgical instrument (1210) has a pin (109) extending through a node of acoustic waveguide (56) and distal shaft portion (62) to fix acoustic waveguide (56) relative to distal shaft portion (62). In turn, pin (109) also longitudinally secures blade (46) in the predetermined alignment position relative to clamp arm (44). Still, the disclosure is not intended to be unnecessarily limited to mechanically fixing blade (46) relative to clamp arm (44). Moreover, such longitudinal adjustments of one or more portions of the acoustic drivetrain along longitudinal axis (61) (*see* FIG. 40B) may be performed in an alternative ultrasonic surgical instrument (not shown) without a clamp arm (not shown) or even to achieve other alignments relative to the acoustic drivetrain without regard for articulation of articulation section (64). The disclosure is thus not intended to be unnecessarily limited to use for maintaining the predetermined alignment between clamp arm (44) and blade (46) as shown and described herein. In any case, like numbers below indicate like features described above in greater detail.

A. Passively Shiftable Transducer Assembly

FIGS. 42-43B show a third example of an ultrasonic surgical instrument (1210) having end effector (16) and a second exemplary shaft assembly (1214) with pin (109) through acoustic waveguide (56) such that blade (46) is longitudinally fixed in the predetermined alignment with clamp arm (44) as discussed above in greater detail.

Ultrasonic surgical instrument (1210) further includes a second exemplary base assembly (1212) constructed similarly to base assembly (12) (*see* FIG. 6), but with a passively shiftable transducer assembly (1254) and a passive system actuator (1236). Shiftable transducer assembly (1254) is movably coupled between housing (18) and passive system actuator (1236) such that passive system actuator (1236) enables shiftable transducer assembly (1254) to be urged proximally or distally along longitudinal axis (61). Thereby, shiftable transducer assembly (1254) accommodates longitudinal movement of acoustic waveguide (56) and blade (46) for maintaining the predetermined alignment. In the present example, pin (109) distally pulls on acoustic waveguide (56) when deflecting end effector away from longitudinal axis (61) such that shiftable transducer assembly (1254) distally pulls shiftable transducer assembly (1254) toward end effector (16) and along longitudinal axis (61). In contrast, pin (109) proximally pushes on acoustic waveguide (56) when deflecting end effector toward longitudinal axis (61) such that shiftable transducer assembly (1254) proximally pushes shiftable transducer assembly (1254) away from end effector (16) and along longitudinal axis (61). Shiftable transducer assembly (1254) and system actuator (1236) are thus referred to herein as "passive" given that shiftable transducer assembly (1254) and system actuator (1236) enable movement rather than providing initiating force for such movement. Although, as will be described below in greater detail, shiftable transducer assembly (1254) and system actuator (1236) may still provide force, such as a reactionary force, for maintaining tension and/or compression on acoustic waveguide (56).

Shiftable transducer assembly (1254) of the present example shown in FIGS. 43A-43B more particularly includes a transducer housing (1270) and a transducer horn (1272) threaded into engagement with acoustic waveguide (56). Housing (18), in one example, has a pair of distal mount seats (1274) and a pair of proximal mount seats (1276) configured to longitudinally capture passive system actuator (1236) while simultaneously allowing for longitudinal movement of transducer housing (1270) and transducer horn (1272) through a central space (1278). Passive system actuator (1236) thereby resiliently and translatably supports shiftable transducer assembly (1254) relative to housing (18) along longitudinal axis (61), although it will be appreciated that the disclosure is not intended to be unnecessarily limited to resilient or translational mounting within housing (18).

More particularly, passive system actuator (1236) of the present example includes an annular base seat (1280) rigidly connected to and extending radially outward from transducer housing (1270) as well as a distal annular spring (1282) and a proximal annular spring (1284). Distal annular spring (1282) seats in compression between annular base seat (1280) and distal mount seats (1274) while proximal annular spring (1284) seats in compression between annular base seat (1280) and proximal mount seats (1276). Distal and proximal mounts seats (1274, 1276) also laterally secure annular base seat (1280) with transducer housing (1270) on longitudinal axis (61). With respect to FIG. 43A, shiftable transducer assembly (1254) is in a proximal position on longitudinal axis (61) while articulation section (64) (*see* FIG. 40A) is in a straight configuration (*see* FIG. 40A). In contrast, with respect to FIG. 43B, shiftable transducer assembly (1254) is in a distal position on longitudinal axis (61) while articulation section (64) (*see* FIG. 40B) is in an articulated configuration (*see* FIG. 40B). In any longitudinal position between the distal and proximal positions, distal and proximal annular springs (1282, 1284) effectively balance force applied by pin (109) *(see* FIG. 42) while annular base seat (1280) translatably supports transducer housing (1270) relative to housing (18) of base assembly (1212). As shown in the present example, distal and proximal annular springs (1282, 1284) are in compression. More particularly, compression of distal annular spring (1282) increases while compression of proximal annular spring (1284) decreases as shiftable transducer assembly (1254) moves from the proximal position toward the distal position. Also, compression of distal annular spring (1282) decreases while compression of proximal annular spring (1284) increases as shiftable transducer assembly (1254) moves from the distal position toward the proximal position.

Distal and proximal annular springs (1282, 1284) are configured to balance annular base seat (1280) with transducer housing (1270) supported therein according to a predetermined balance in any longitudinal position for accommodating movement of acoustic waveguide (56) resulting from articulation of articulation section (64) (*see* FIG. 40A). In one example, distal and proximal annular springs (1282, 1284) balance acoustic waveguide (56) in tension between the proximal and distal positions. In another example, distal and proximal annular springs (1282, 1284) balance acoustic waveguide (56) in compression between the proximal and distal positions. In still another example, distal and proximal annular springs (1282, 1284) balance acoustic waveguide (56) in compression toward the proximal position and in tension toward the distal position with a neutral, non-compression and non-tension state therebetween. In still yet another example, distal and proximal annular springs (1282, 1284) balance acoustic waveguide (56) in tension toward the proximal position and in compression toward the distal position with another neutral, non-compression and non-tension state therebetween. The disclosure is thus not intended to be unnecessarily limited to maintaining one or more portions of the acoustic drivetrain, such acoustic waveguide (56), in a particular state of compression or tension.

In use, with respect to FIGS. 6 and 42-43B, linear system actuators (36e, 36f) urge articulation bands (74) (*see* FIG. 40B) to direct movement of articulation section (64) for deflecting end effector (16) relative to longitudinal axis (61). Articulation section (64) articulates from the straight configuration toward the articulated configuration such that pin (109) distally pulls acoustic waveguide (56) with shiftable transducer assembly (1254) from the proximal position toward the distal position to maintain the predetermined alignment between blade (46) and clamp arm (44). In this respect, movement of shiftable transducer assembly (1254) is dependent upon articulation of articulation section (64). Annular base seat (1280) of passive system actuator (1236) supports transducer housing (1270) and transducer horn (1272) as shiftable transducer assembly (1254) translates from the proximal position toward the distal position. In addition, annular base seat (1280) compresses distal annular spring (1282) against distal mount seats (1274), while proximal annular spring (1284) expands, distally urging annular base seat (1280) toward distal mount seats (1274). Distal and proximal annular springs (1282, 1284) continue to balance shiftable transducer assembly (1254) and acoustic waveguide (56) with pin (109) as articulation section (64) is positioned in the articulated configuration during use. Thus, blade (46) remains in the same predetermined alignment with clamp arm (44) before, during, and after articulation of articulation section (64). Of course, any alignment may be desired, and the disclosure is not intended to be limited to the alignment of blade (46) shown and described in the present example. While the present example describes movement of articulation section (64) from the straight configuration toward the articulated configuration, it will be appreciated that shiftable transducer assembly (1254) and associated components will move in opposite directions from those discussed above when moving articulation section (64) from the articulated configuration toward the straight configuration. Furthermore, in another example, articulation of articulation section (64) may be controlled by an operator via a handle assembly (not shown), such as by a knob (not shown) operatively connected to articulation section (64), rather than a robotic drive (not shown). The disclosure is thus not intended to be unnecessarily limited to use with base assembly (1212) as shown and described herein.

B. Actively Shiftable Transducer Assembly

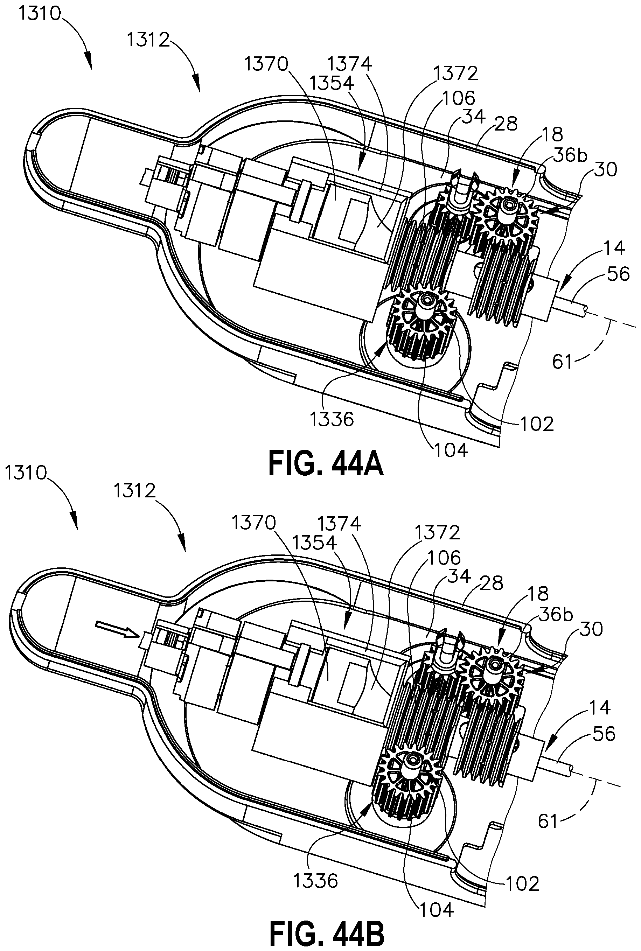

FIGS. 44A-44B show a fourth example of an ultrasonic surgical instrument (1310) having end effector (16) (*see* FIG. 41A) and shaft assembly (14) without pin (109) (*see* FIG. 42) through acoustic waveguide (56) such that blade (46) may or may not be longitudinally maintained in the predetermined alignment with clamp arm (44). Ultrasonic surgical instrument (1310) further includes a third exemplary base assembly (1312) constructed similarly to base assembly (12) (*see* FIG. 6), but with an actively shiftable transducer assembly (1354) and an active system actuator (1336). Shiftable transducer assembly (1354) is movably coupled between housing (18) and active system actuator (1336) such that active system actuator (1336) urges shiftable transducer assembly (1354) proximally or distally along longitudinal axis (61). In one example, active system actuator (1336) keys movement of shiftable transducer assembly (1354) to accommodate longitudinal movement of acoustic waveguide (56) and blade (46) (*see* FIGS. 41A) for maintaining the predetermined alignment during articulation. Either shaft assembly (14) or shaft assembly (1214) (*see* FIG. 42) may be incorporated into ultrasonic surgical instrument (1310) in such an example. In another example, active system actuator (1336) directs movement of shiftable transducer assembly (1354) to position blade (46) in any longitudinal position relative to shaft assembly (14) and/or clamp arm (44) as desired by the operator. The disclosure is thus not intended to be unnecessarily limited to acoustic waveguide (56) being movable or fixed, such as via pin (109). Shiftable transducer assembly (1354) and system actuator (1336) are thus referred to herein as "active" given that shiftable transducer assembly (1354) and system actuator (1336) initiate movement by force rather than simply supporting such movement.

Shiftable transducer assembly (1354) of the present example shown in FIGS. 44A-44B more particularly includes a transducer housing (1370) and a transducer horn (1372) threaded into engagement with acoustic waveguide (56). Active system actuator (1336) of the present example includes gear-rack mechanism (102) with rotatable drive gear (104) and translatable rack gear (106) discussed above in greater detail as well as a transducer coupler (1374) rigidly extending in the proximal direction from rack gear (106). Transducer coupler (1374) receives transducer housing (1370) to longitudinally secure transducer housing (1370) and transducer horn (1372) relative to rack gear (106) distally extending therefrom while laterally supporting transducer housing (1370) and transducer horn (1372). Rotatable drive gear (104) directly engages with rack gear (106) in the present example to selectively translate shiftable transducer assembly (1354) as desired.

In use, with respect to FIGS. 41A and 44A-44B, drive gear (104) is selectively rotated via robotic drive (not shown) to linearly translate rack gear (106) along longitudinal axis (61) as desired. In turn, transducer coupler (1374) urges shiftable transducer assembly (1354) distally or proximally along longitudinal axis (61) thereby translating acoustic waveguide (56) and blade (46) as desired. In one example, active system actuator (1336) keys movement of shiftable transducer assembly (1354) to accommodate longitudinal movement of acoustic waveguide (56) and blade (46) for maintaining the predetermined alignment during articulation. Alternatively or in addition, active system actuator (1336) directs movement of shiftable transducer assembly (1354) to position blade (46) in any longitudinal position relative to shaft assembly (14) and/or clamp arm (44) as desired by the operator. In this respect, movement of shiftable transducer assembly (1354) is independent upon articulation of articulation section (64) such that shiftable transducer assembly (1354) may be moved with or without articulating articulation section (64). While articulation of articulation section (64) and/or shiftable transducer assembly (1354) may be controlled by a robotic drive (not shown) as discussed in the present example, an operator may alternatively control articulation of articulation section (64) and/or shiftable transducer assembly (1354) via a handle assembly (not shown), such as by a knob (not shown) operatively connected to articulation section (64) and/or shiftable transducer assembly (1354). The disclosure is thus not intended to be unnecessarily limited to use with base assembly (1312) as shown and described herein.

VI. Miscellaneous

Any one or more of the teaching, expressions, embodiments, examples, etc. described herein may be combined with any one or more of the teachings, expressions, embodiments, examples, etc. described in U.S. Pat. App. No. 16/556,625, entitled "Ultrasonic Surgical Instrument with Axisymmetric Clamping," filed on even date herewith; U.S. Pat. App. No. 16/556,635, entitled "Ultrasonic Blade and Clamp Arm Alignment Features," filed on even date herewith; and/or U.S. Pat. App. No. 16/556,727, entitled " Rotatable Linear Actuation Mechanism," filed on even date herewith.

It should be understood that any of the versions of instruments described herein may include various other features in addition to or in lieu of those described above. By way of example only, in addition to the teachings above, it should be understood that the instruments described herein may be constructed and operable in accordance with at least some of the teachings of U.S. Pat. No. 5,322,055; U.S. Pat. No. 5,873,873; U.S. Pat. No. 5,980,510; U.S. Pat. No. 6,325,811; U.S. Pat. No. 6,773,444; U.S. Pat. No. 6,783,524; U.S. Pat. No. 9,095,367; U.S. Pub. No. 2006/0079874; U.S. Pub. No. 2007/0191713; U.S. Pub. No. 2007/0282333; U.S. Pub. No. 2008/0200940; U.S. Pat. No. 8,623,027, issued January 7, 2014; U.S. Pat. No. 9,023,071, issued May 5, 2015; U.S. Pat. No. 8,461,744, issued June 11, 2013; U.S. Pat. No. 9,381,058, issued July 5, 2016; U.S. Pub. No. 2012/0116265; U.S. Pat. No. 9,393,037, issued July 19, 2016; U.S. Pat. No. 10,172,636, issued January 8, 2019; and/or U.S. Pat. App. No. 61/410,603. The disclosures of each of the foregoing patents, publications, and applications are referred to herein. It should also be understood that the instruments described herein may have various structural and functional similarities with the HARMONIC ACE® Ultrasonic Shears, the HARMONIC WAVE® Ultrasonic Shears, the HARMONIC FOCUS® Ultrasonic Shears, and/or the HARMONIC SYNERGY® Ultrasonic Blades. Furthermore, the instruments described herein may have various structural and functional similarities with the devices taught in any of the other references that are referred to herein.

To the extent that there is some degree of overlap between the teachings of the references cited herein, the HARMONIC ACE® Ultrasonic Shears, the HARMONIC WAVE® Ultrasonic Shears, the HARMONIC FOCUS® Ultrasonic Shears, and/or the HARMONIC SYNERGY® Ultrasonic Blades, and the teachings herein relating to the instruments described herein, there is no intent for any of the description herein to be presumed as admitted prior art. Several teachings herein will in fact go beyond the scope of the teachings of the references cited herein and the HARMONIC ACE® Ultrasonic Shears, the HARMONIC WAVE® Ultrasonic Shears, the HARMONIC FOCUS® Ultrasonic Shears, and the HARMONIC SYNERGY® Ultrasonic Blades.

Versions of the devices described above may have application in conventional medical treatments and procedures conducted by a medical professional, as well as application in robotic-assisted medical treatments and procedures. By way of example only, various teachings herein may be readily incorporated into another example of a robotic surgical system, and those of ordinary skill in the art will recognize that various teachings herein may be readily combined with various teachings of any of the following: U.S. Pat. No. 8,844,789, entitled "Automated End Effector Component Reloading System for Use with a Robotic System," issued Sep. 30, 2014, the disclosure of which is referred to herein; U.S. Pat. No. 8,820,605, entitled "Robotically-Controlled Surgical Instruments," issued Sep. 2, 2014, the disclosure of which is referred to herein; U.S. Pat. No. 8,616,431, entitled "Shiftable Drive Interface for Robotically-Controlled Surgical Tool," issued Dec. 31, 2013, the disclosure of which is referred to herein; U.S. Pat. No. 8,573,461, entitled "Surgical Stapling Instruments with Cam-Driven Staple Deployment Arrangements," issued Nov. 5, 2013, the disclosure of which is referred to herein; U.S. Pat. No. 8,602,288, entitled "Robotically-Controlled Motorized Surgical End Effector System with Rotary Actuated Closure Systems Having Variable Actuation Speeds," issued Dec. 10, 2013, the disclosure of which is referred to herein; U.S. Pat. No. 9,301,759, entitled "Robotically-Controlled Surgical Instrument with Selectively Articulatable End Effector," issued Apr. 5, 2016, the disclosure of which is referred to herein; U.S. Pat. No. 8,783,541, entitled "Robotically-Controlled Surgical End Effector System," issued Jul. 22, 2014, the disclosure of which is referred to herein; U.S. Pat. No. 8,479,969, entitled "Drive Interface for Operably Coupling a Manipulatable Surgical Tool to a Robot," issued Jul. 9, 2013; U.S. Pat. No. 8,800,838, entitled "Robotically-Controlled Cable-Based Surgical End Effectors," issued Aug. 12, 2014, the disclosure of which is referred to herein; and/or U.S. Pat. No. 8,573,465, entitled "Robotically-Controlled Surgical End Effector System with Rotary Actuated Closure Systems," issued Nov. 5, 2013, the disclosure of which is referred to herein.

Versions described above may be designed to be disposed of after a single use, or they can be designed to be used multiple times. Versions may, in either or both cases, be reconditioned for reuse after at least one use.

Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present disclosure.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments, further adaptations of the systems described herein may be accomplished by appropriate modifications by one of ordinary skill in the art without departing from the scope of the present invention defined in the appended claims. Several of such potential modifications have been mentioned, and others will be apparent to those skilled in the art. For instance, the examples, embodiments, geometrics, materials, dimensions, ratios, and the like discussed above are illustrative and are not required. Accordingly, the scope of the present invention should be considered in terms of the following claims and is understood not to be limited to the details of structure and operation shown and described in the specification and drawings.

**Claims**

1. An acoustic waveguide (556) for an ultrasonic surgical instrument, comprising:

   (a) a proximal waveguide body portion (560) defining a longitudinal axis (561);
   (b) a distal waveguide body portion (562) having an ultrasonic blade (546) distally projecting therefrom; and
   (c) an articulation body portion (564) extending between the proximal and distal waveguide body portions, wherein the articulation body comprises a first flexible member (558) and a second flexible member, wherein the first and second flexible members are configured to flex in a full 360-degree range of radial directions through a respective full 360-degree range of radial planes such that the articulation body portion is configured to flex in at least a first direction and a second direction to thereby deflect the ultrasonic blade relative to the longitudinal axis and through a first plane and through a second plane respectively,

and

wherein the second direction is different than the first direction such that the second plane is different than the first plane for multiplanar deflection of the ultrasonic blade relative to the longitudinal axis;

wherein the first flexible member is a flexible member configured to receive an acoustic vibration from the proximal waveguide body portion, and communicate the acoustic vibration from the proximal waveguide body portion to the distal waveguide body portion for driving the ultrasonic blade with the acoustic vibration; and wherein the first flexible member has a set of predetermined properties and the acoustic vibration has a longitudinal vibrational component and a transverse vibrational component, and wherein the set of predetermined properties of the flexible member are configured to uncouple the longitudinal vibrational component from the transverse vibrational component;

wherein the first flexible member is a first flexible wire (566) and wherein the first flexible wire has a wire cross-sectional radius, the proximal waveguide body portion has a proximal waveguide radius, and the distal waveguide body portion has a distal waveguide radius, and wherein the wire cross-sectional radius is smaller than the proximal and distal waveguide radii, and wherein the second flexible member is a second flexible wire.

2. The acoustic waveguide of claim 1, wherein the first and second flexible members are positioned and centered on anti-nodes of the waveguide.

3. The acoustic waveguide of claim 1, wherein the first flexible member is affixed to the proximal waveguide body portion at a proximal component joint, and wherein the second flexible member is affixed to the distal waveguide body portion at a distal component joint.

4. The acoustic waveguide of claim 1, wherein the ultrasonic blade extends along a blade axis and has a blade body defining a circumferential blade profile about the blade axis, and wherein the ultrasonic blade includes a backcutting edge longitudinally extending along the blade body such that the circumferential blade profile is circular about a majority of the circumferential blade profile and configured to engage a clamp pad.

5. The acoustic waveguide of claim 4, wherein the blade body has a distal blade portion that tapers to a distal blade tip, and wherein at least a majority of the backcutting edge longitudinally extends along the distal blade portion that tapers to the distal blade tip.

6. An ultrasonic surgical instrument, comprising:

(a) an end effector (16) including an ultrasonic blade;
(b) a body assembly; and
(c) a shaft assembly (14) having a first articulation section and longitudinally extending from the body assembly to the end effector, including the acoustic waveguide of claim 1, wherein;

the proximal waveguide body portion is positioned proximally from the first articulation section;
the distal waveguide body portion is positioned distally from the first articulation section and is in acoustic communication with the ultrasonic blade distally projecting therefrom; and

an articulation body portion extending through the first articulation section.

7. The ultrasonic surgical instrument of claim 6, wherein the first articulation section of the shaft assembly is configured to limit the first flexible member to a predetermined maximum bend radius.

8. The ultrasonic surgical instrument of claim 6, wherein the shaft assembly further includes a second articulation section, and wherein the first and second flexible members are positioned in the first and second articulation sections.

9. The ultrasonic surgical instrument of claim 6, wherein the body assembly further includes a robotic driven interface operatively connected to the articulation body portion and configured to connect to a robotic drive for selectively directing flexing of the articulation body portion in the first or second directions.

**Patentansprüche**

1. Akustischer Wellenleiter (556) für ein chirurgisches Ultraschallinstrument, umfassend:

   (a) einen proximalen Wellenleiterkörperabschnitt (560), der eine Längsachse (561) definiert;

   (b) einen distalen Wellenleiterkörperabschnitt (562), der eine distal davon vorstehende Ultraschallklinge (546) aufweist; und

   (c) einen Gelenkkörperabschnitt (564), der sich zwischen dem proximalen und distalen Wellenleiterkörperabschnitt erstreckt, wobei der Gelenkkörper ein erstes flexibles Element (558) und ein zweites flexibles Element umfasst, wobei das erste und das zweite flexible Element konfiguriert sind, um sich in einem vollen 360-Grad-Bereich radialer Richtungen durch einen jeweiligen vollen 360-Grad-Bereich radialer Ebenen zu biegen, so dass der Gelenkkörperabschnitt konfiguriert ist, um sich in mindestens eine erste Richtung und eine zweite Richtung zu biegen, um dadurch die Ultraschallklinge relativ zur Längsachse und jeweils durch eine erste Ebene und durch eine zweite Ebene abzulenken,

   und

   wobei sich die zweite Richtung von der ersten Richtung unterscheidet, so dass sich die zweite Ebene von der ersten Ebene für eine multiplanare Ablenkung der Ultraschallklinge relativ zur Längsachse unterscheidet;

   wobei das erste flexible Element ein flexibles Element ist, das konfiguriert ist, um eine akustische Schwingung vom proximalen Wellenleiterkörperabschnitt zu empfangen und die akustische Schwingung vom proximalen Wellenleiterkörperabschnitt zum distalen Wellenleiterkörperabschnitt zu kommunizieren, um die Ultraschallklinge mit der akustischen Schwingung anzutreiben; und

   wobei das erste flexible Element einen Satz von vorbestimmten Eigenschaften aufweist und die akustische Schwingung eine Längsschwingungskomponente und eine Querschwingungskomponente aufweist, und wobei der Satz von vorbestimmten Eigenschaften des flexiblen Elements konfiguriert ist, um die Längsschwingungskomponente von der Querschwingungskomponente zu entkoppeln;

   wobei das erste flexible Element ein erster flexibler Draht (566) ist und wobei der erste flexible Draht einen Drahtquerschnittsradius aufweist, der proximale Wellenleiterkörperabschnitt einen proximalen Wellenleiterradius aufweist und der distale Wellenleiterkörperabschnitt einen distalen Wellenleiterradius aufweist und wobei der Drahtquerschnittsradius kleiner ist als die proximalen und distalen Wellenleiterradien, und

   wobei das zweite flexible Element ein zweiter flexibler Draht ist.

2. Akustischer Wellenleiter nach Anspruch 1, wobei das erste und das zweite flexible Element auf den Schwingungsbäuchen des Wellenleiters positioniert und zentriert sind.

3. Akustischer Wellenleiter nach Anspruch 1, wobei das erste flexible Element an einer proximalen Komponentenverbindung am proximalen Wellenleiterkörperabschnitt befestigt ist und wobei das zweite flexible Element an einer distalen Komponentenverbindung am distalen Wellenleiterkörperabschnitt befestigt ist.

4. Akustischer Wellenleiter nach Anspruch 1, wobei sich die Ultraschallklinge entlang einer Klingenachse erstreckt und einen Klingenkörper aufweist, der ein um die Klingenachse herum verlaufendes Klingenprofil definiert, und wobei die Ultraschallklinge eine Rückschneide einschließt, die sich in Längsrichtung entlang des Klingenkörpers erstreckt, so dass das umlaufende Klingenprofil um einen Großteil des umlaufenden Klingenprofils kreisförmig ist und zum Eingreifen in eine Klemmauflage konfiguriert ist.

5. Akustischer Wellenleiter nach Anspruch 4, wobei der Klingenkörper einen distalen Klingenabschnitt aufweist, der sich zu einer distalen Klingenspitze verjüngt, und wobei sich mindestens ein Großteil der Rückschneide in Längsrichtung entlang des distalen Klingenabschnitts erstreckt, der sich zu der distalen Klingenspitze verjüngt.

6. Chirurgisches Ultraschallinstrument, umfassend:

   (a) einen Endeffektor (16), der eine Ultraschallklinge einschließt;

   (b) eine Körperbaugruppe; und

   (c) eine Schaftbaugruppe (14), die einen ersten Gelenkabschnitt aufweist und sich in Längsrichtung von der Körperbaugruppe zum Endeffektor erstreckt, einschließlich des akustischen Wellenleiters nach Anspruch 1, wobei:

   der proximale Wellenleiterkörperabschnitt proximal vom ersten Gelenkabschnitt positioniert ist;

   der distale Wellenleiterkörperabschnitt distal vom ersten Gelenkabschnitt positioniert ist und in akustischer Kommunikation mit der distal davon vorstehenden Ultraschallklinge steht; und
sich ein Gelenkkörperabschnitt durch den ersten Gelenkabschnitt erstreckt.

**7.** Chirurgisches Ultraschallinstrument nach Anspruch 6, wobei der erste Gelenkabschnitt der Schaftbaugruppe konfiguriert ist, um das erste flexible Element auf einen vorgegebenen maximalen Biegeradius zu begrenzen.

**8.** Chirurgisches Ultraschallinstrument nach Anspruch 6, wobei die Schaftbaugruppe ferner einen zweiten Gelenkabschnitt einschließt und wobei das erste und das zweite flexible Element in dem ersten und dem zweiten Gelenkabschnitt positioniert sind.

**9.** Chirurgisches Ultraschallinstrument nach Anspruch 6, wobei die Körperbaugruppe ferner eine robotergesteuerte Schnittstelle einschließt, die funktional mit dem Gelenkkörperabschnitt verbunden ist und konfiguriert ist, um mit einem Roboterantrieb verbunden werden zu können, um die Biegung des Gelenkkörperabschnitts selektiv in die erste oder zweite Richtung zu lenken.

**Revendications**

**1.** Guide d'ondes acoustiques (556) pour un instrument chirurgical à ultrasons, comprenant :

(a) une partie corps de guide d'ondes proximale (560) définissant un axe longitudinal (561) ;
(b) une partie corps de guide d'ondes distale (562) ayant une lame à ultrasons (546) faisant saillie de manière distale à partir de celle-ci ; et
(c) une partie corps d'articulation (564) s'étendant entre les parties corps de guide d'ondes proximale et distale, dans lequel le corps d'articulation comprend un premier élément flexible (558) et un second élément flexible, dans lequel les premier et second éléments flexibles sont conçus pour fléchir dans une plage complète de 360 degrés de directions radiales à travers une plage complète de 360 degrés de plans radiaux respectifs de sorte que la partie corps d'articulation est conçue pour fléchir dans au moins une première direction et une seconde direction pour dévier ainsi la lame à ultrasons par rapport à l'axe longitudinal et à travers un premier plan et à travers un second plan, respectivement,

et

dans lequel la seconde direction est différente de la première direction, de sorte que le second plan est différent du premier plan pour une déviation multiplanaire de la lame à ultrasons par rapport à l'axe longitudinal ;
dans lequel le premier élément flexible est un élément flexible conçu pour recevoir une vibration acoustique depuis la partie corps de guide d'ondes proximale, et communiquer la vibration acoustique depuis la partie corps de guide d'ondes proximale à la partie corps de guide d'ondes distale pour entraîner la lame à ultrasons avec la vibration acoustique ; et
dans lequel le premier élément flexible a un ensemble de propriétés prédéterminées et la vibration acoustique a une composante de vibration longitudinale et une composante de vibration transversale, et dans lequel l'ensemble des propriétés prédéterminées de l'élément flexible sont conçues pour désaccoupler la composante de vibration longitudinale de la composante de vibration transversale ;
dans lequel le premier élément flexible est un premier fil flexible (566) et dans lequel le premier fil flexible a un rayon transversal de fil, la partie corps de guide d'ondes proximale a un rayon de guide d'ondes proximal, et la partie corps de guide d'ondes distale a un rayon de guide d'ondes distal, et dans lequel le rayon transversal de fil est plus petit que les rayons de guide d'ondes proximal et distal, et
dans lequel le second élément flexible est un second fil flexible.

**2.** Guide d'ondes acoustiques selon la revendication 1, dans lequel les premier et second éléments flexibles sont positionnés et centrés sur des ventres du guide d'ondes.

**3.** Guide d'ondes acoustiques selon la revendication 1, dans lequel le premier élément flexible est fixé à la partie corps de guide d'ondes proximale au niveau d'une articulation de composant proximal, et dans lequel le second élément flexible est fixé à la partie corps de guide d'ondes distale au niveau d'une articulation de composant distal.

**4.** Guide d'ondes acoustiques selon la revendication 1, dans lequel la lame à ultrasons s'étend le long d'un axe de lame et a un corps de lame définissant un profil de lame circonférentiel autour de l'axe de lame, et dans lequel la lame à ultrasons comporte un bord de coupe arrière s'étendant longitudinalement le long du corps de lame de sorte que le profil de lame circonférentiel est circulaire autour d'une majorité du profil de lame circonférentiel et conçu pour venir en prise avec un tampon de serrage.

**5.** Guide d'ondes acoustiques selon la revendication 4, dans lequel le corps de lame a une partie distale de lame qui se rétrécit jusqu'à une pointe distale de lame, et dans lequel au moins une majorité du bord de coupe arrière s'étend longitudinalement le long de la partie distale de lame qui se rétrécit jusqu'à la pointe distale de lame.

6. Instrument chirurgical à ultrasons, comprenant :

(a) un effecteur (16) comportant une lame à ultrasons ;
(b) un ensemble corps ; et
(c) un ensemble arbre (14) ayant une première section d'articulation et s'étendant longitudinalement depuis l'ensemble corps jusqu'à l'effecteur, comportant le guide d'ondes acoustiques selon la revendication 1, dans lequel ;

la partie corps de guide d'ondes proximale est positionnée de manière proximale par rapport la première section d'articulation ;
la partie corps de guide d'ondes distale est positionnée de manière distale par rapport à la première section d'articulation et est en communication acoustique avec la lame à ultrasons qui fait saillie de manière distale à partir de celle-ci ; et
une partie corps d'articulation s'étendant à travers la première section d'articulation.

7. Instrument chirurgical à ultrasons selon la revendication 6, dans lequel la première section d'articulation de l'ensemble arbre est conçue pour limiter le premier élément flexible à un rayon de courbure maximal prédéterminé.

8. Instrument chirurgical à ultrasons selon la revendication 6, dans lequel l'assemblage arbre comporte en outre une seconde section d'articulation, et dans lequel les premier et second éléments flexibles sont positionnés dans les première et seconde sections d'articulation.

9. Instrument chirurgical à ultrasons selon la revendication 6, dans lequel l'ensemble corps comporte en outre une interface à entraînement robotique connectée de manière opérationnelle à la partie corps d'articulation et configurée pour se connecter à un entraînement robotique afin de diriger sélectivement la flexion de la partie corps d'articulation dans les première ou seconde directions.

**FIG. 1**

EP 4 140 420 B1

FIG. 2

EP 4 140 420 B1

**FIG. 3A**

**FIG. 3B**

FIG. 4A

FIG. 4B

28

FIG. 5

**FIG. 6**

**FIG. 7**

EP 4 140 420 B1

**FIG. 8**

EP 4 140 420 B1

**FIG. 9**

**FIG. 10**

**FIG. 11**

**FIG. 12**

FIG. 14

FIG. 16

FIG. 13

FIG. 15

FIG. 17

FIG. 18

FIG. 19

FIG. 20

EP 4 140 420 B1

FIG. 22

FIG. 24

FIG. 21

FIG. 23

37

**FIG. 25**

EP 4 140 420 B1

**FIG. 26**

**FIG. 27**

561

560

572

564

29

574

558

574

566

562

29

546

572

576

**FIG. 28**

572

574

r

566

561

**FIG. 29**

**FIG. 30A**

**FIG. 30B**

FIG. 31

FIG. 32

**FIG. 33**

**FIG. 34**

**FIG. 35A**

**FIG. 35B**

**FIG. 36**

**FIG. 37**

FIG. 38

FIG. 39

## FIG. 40A

## FIG. 40B

**FIG. 41A**

**FIG. 41B**

**FIG. 42**

**FIG. 43A**

**FIG. 43B**

**FIG. 44A**

**FIG. 44B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9095367 B2
- US 20170105757 A1
- US 20140005681 A1
- US 9402682 B
- US 9393037 B
- US 9095367 B
- US 10034683 B
- US 20110087212 A
- US 556625
- US 556635
- US 556727
- US 5322055 A
- US 5873873 A
- US 5980510 A
- US 6325811 B
- US 6773444 B
- US 6783524 B
- US 20060079874 A
- US 20070191713 A

- US 20070282333 A
- US 20080200940 A
- US 8623027 B
- US 9023071 B
- US 8461744 B
- US 9381058 B
- US 20120116265 A
- US 10172636 B
- US 61410603
- US 8844789 B
- US 8820605 B
- US 8616431 B
- US 8573461 B
- US 8602288 B
- US 9301759 B
- US 8783541 B
- US 8479969 B
- US 8800838 B
- US 8573465 B